# EUROPEAN PATENT APPLICATION

(11) **EP 3 660 053 A1**
(43) Date of publication of application: **03.06.2020**
(21) Application number: 18208613.2
(22) Date of filing: 27.11.2018
(51) Int. Cl.: C07K 19/00, C12N 15/82

(54) **MULTIPROTEIN EXPRESSION SYSTEMS FOR PEST CONTROL**

(71) Applicant: Fraunhofer-Gesellschaft zur Förderung der angewandten Forschung e.V., 80686 München (DE); Justus-Liebig-Universität Gießen, 35390 Gießen (DE)
(72) Inventor: Schetelig, Marc, 35394 Gießen (DE); Schwirz, Jonas, 35394 Gießen (DE); Yan, Ying, 35394 Gießen (DE)
(74) Representative: Wichmann, Hendrik

(57) **Abstract**

The application describes a polycistronic expression cassette comprising two or more coding sequences each separated by a coding sequence encoding a self-cleaving peptides sequence. Self-cleaving peptides cause a ribsosomal skipping event allowing a polycistronic translation of two or more coding sequences under the control of a single promoter element. One or more of said coding sequences encode one or more lethal gene product(s) making this expression cassette a lethality system. The expression cassette is under the control of a single repressible promoter. The application also describes an expression construct, which further comprises a second expression cassette. The second expression cassette optionally encodes one or more male determining gene(s). Also, the application describes a transgenic insect comprising this polycistronic expression cassette or expression construct. The transgenic insect is a crop pest insect or a disease transmitting insect. The application also describes the use of a transgenic insect according to the invention for controlling reproduction in an insect population of interest. Furthermore, the application provides a method of controlling reproduction in an insect population of interest.

SEQUENCE LISTING

<110>  Fraunhofer-Gesellschaft zur Förderung der angewandten
       Forschung e.V.; Justus-Liebig-Universität Gießen

<120>  Multiprotein expression systems for pest control

<130>  EPA-144 652

<160>  30

<170>  PatentIn version 3.5

<210>  1
<211>  38
<212>  PRT
<213>  Drosophila suzukii

<400>  1

Ser Arg His Arg Arg His Arg Gln Arg Ser Arg Ser Arg Asn Arg Ser
1               5                   10                  15

Arg Ser Arg Ser Ser Glu Arg Arg His Arg Ala Arg Ser Pro His Arg
            20                  25                  30

Tyr Asn Pro Pro Pro Lys
        35

<210>  2
<211>  17
<212>  PRT
<213>  Artificial Sequence

<220>
<223>  a peptide-tag consisting of a polybasic Lysine rich motif
       combined with a CAAX motif for membrane localization

<400>  2

Lys Asp Gly Lys Lys Lys Lys Lys Lys Ser Lys Thr Lys Cys Val Ile
1               5                   10                  15

Met

<210>  3
<211>  23
<212>  PRT
<213>  Artificial Sequence

<220>
<223>  2A peptide sequence derived from the Thosea asigna virus (TaV)

<400>  3

Ala Ile Ala Arg Ala Glu Gly Arg Gly Ser Leu Leu Thr Cys Gly Asp
1               5                   10                  15

Val Glu Glu Asn Pro Gly Pro
            20

<210>  4
<211>  22
<212>  PRT
<213>  Artificial Sequence

<220>
<223>  2A peptide sequences from Drosophila C virus (DrosCV)

<400>  4

Ala Ile Ala Ala Ala Arg Gln Met Leu Leu Leu Leu Ser Gly Asp Val
1               5                   10                  15

Glu Thr Asn Pro Gly Pro
            20

<210>  5
<211>  61
<212>  PRT
<213>  Artificial Sequence

<220>
<223>  2A sequences derived from the foot and mouth disease virus

<400>  5

Ala Ile Ala Val Thr Glu Leu Leu Tyr Arg Met Lys Arg Ala Glu Thr
1               5                   10                  15

Tyr Cys Pro Arg Pro Leu Leu Ala Ile His Pro Thr Glu Ala Arg His
            20                  25                  30

Lys Gln Lys Ile Val Ala Pro Val Lys Gln Thr Leu Asn Phe Asp Leu
        35                  40                  45

Leu Lys Leu Ala Gly Asp Val Glu Ser Asn Pro Gly Pro
    50                  55                  60

<210>  6
<211>  43
<212>  PRT
<213>  Artificial Sequence

<220>
<223>  artificial 2A sequences derived from the foot and mouth disease
       virus

<400>  6

Ala Ile Ala Leu Leu Ala Ile His Pro Thr Glu Ala Arg His Lys Gln
1               5                   10                  15

Lys Ile Val Ala Pro Val Lys Gln Thr Leu Asn Phe Asp Leu Leu Lys
            20                  25                  30

Leu Ala Gly Asp Val Glu Ser Asn Pro Gly Pro
        35                  40

<210>  7
<211>  19
<212>  DNA
<213>  Artificial Sequence

<220>
<223>  primer MFS8

<400>  7
aagattagcg acgctgctg                                                    19

<210>  8
<211>  36
<212>  DNA
<213>  Artificial Sequence

<220>
<223>  primer P39

<400>  8
ctgcgcgcga tcgatagttc tttgcctgta tttcag                                 36

<210>  9
<211>  43
<212>  DNA
<213>  Artificial Sequence

<220>
<223>  primer P40

<400>  9
ttacttttat ctaatctaga catagctcta taagatgtgc tcc                         43

<210>  10
<211>  23
<212>  DNA
<213>  Artificial Sequence

<220>
<223>  primer P180

<400>  10
ctacgtggaa gtggaagaag aag                                               23

<210>  11
<211>  20
<212>  DNA
<213>  Artificial Sequence

<220>
<223>  primer P191

<400>  11
cagttgcaag ttgacactgg                                                    20


<210>  12
<211>  30
<212>  DNA
<213>  Artificial Sequence

<220>
<223>  primer P225

<400>  12
tatccgcgga tggtgagcaa gggcgaggag                                         30


<210>  13
<211>  44
<212>  DNA
<213>  Artificial Sequence

<220>
<223>  primer P226

<400>  13
tatgcggccg cctacgcgat cgcctttggc ggcggattgt accg                         44


<210>  14
<211>  19
<212>  DNA
<213>  Artificial Sequence

<220>
<223>  primer P227

<400>  14
tgccgtcctc cttcttaac                                                     19


<210>  15
<211>  21
<212>  DNA
<213>  Artificial Sequence

<220>
<223>  primer P260

<400>  15
agcaggcaca gaaggcatcg c                                                  21


<210>  16
<211>  38
<212>  DNA
<213>  Artificial Sequence

<220>
<223>  primer P261

<400>  16
atgccttctg tgcctgctct tgtacagctc gtccatgc                              38


<210>  17
<211>  31
<212>  DNA
<213>  Artificial Sequence

<220>
<223>  primer P262

<400>  17
tatccgcgga tggcctcctc cgaggacgtc a                                     31


<210>  18
<211>  39
<212>  DNA
<213>  Artificial Sequence

<220>
<223>  primer P263

<400>  18
atgccttctg tgcctgctca ggaacaggtg gtggcggcc                             39


<210>  19
<211>  37
<212>  DNA
<213>  Artificial Sequence

<220>
<223>  primer P264

<400>  19
acatccgcgg gggcccatgg cctcctccga ggacgtc                               37


<210>  20
<211>  86
<212>  DNA
<213>  Artificial Sequence

<220>
<223>  primer P265

<400>  20
   acatgcggcc gcttacatga taacacactt ggtttttgac ttcttcttct ttttcttacc       60
   atccttcagg aacaggtggt ggcggc                                           86


<210>  21
<211>  38
<212>  DNA
<213>  Artificial Sequence

<220>
<223>  primer P266

<400>  21

acatccgcgg gggcccatgg tgagcaaggg cgaggagc 38

<210> 22
<211> 90
<212> DNA
<213> Artificial Sequence

<220>
<223> primer P267

<400> 22
   acatgcggcc gcttacatga taacacactt ggtttttgac ttcttcttct ttttcttacc 60
   atccttcttg tacagctcgt ccatgccgag 90

<210> 23
<211> 35
<212> DNA
<213> Artificial Sequence

<220>
<223> primer P362

<400> 23
acatgcgatc gcgatggtga gcaagggcga ggagc 35

<210> 24
<211> 34
<212> DNA
<213> Artificial Sequence

<220>
<223> primer P363

<400> 24
acatgcgatc gcgatggcct cctccgagga cgtc 34

<210> 25
<211> 50
<212> DNA
<213> Artificial Sequence

<220>
<223> primer P392

<400> 25
aactagtcgc gaggcctgca gatctatggt atatatacac atattcgatc 50

<210> 26
<211> 44
<212> DNA
<213> Artificial Sequence

<220>
<223> primer P393

<400> 26
cttcttcttc cacttccacg taggcctcct ccgaggacgt catc 44

<210>  27
<211>  46
<212>  DNA
<213>  Artificial Sequence

<220>
<223>  primer P394

<400>  27
ggctgattat gatctagagt cgcggccgcc tacgcgatcg cctttg                    46


<210>  28
<211>  32
<212>  DNA
<213>  Artificial Sequence

<220>
<223>  primer P395

<400>  28
atgccctgag gttgtcgccg gaacgcagcg ac                                   32


<210>  29
<211>  51
<212>  DNA
<213>  Artificial Sequence

<220>
<223>  primer P396

<400>  29
tagcagatct ggccggccgc ggacgcgttg gatccccgat ctgcattttg g             51


<210>  30
<211>  1345
<212>  DNA
<213>  Ceratitis capitata

<400>  30
gtaattttaa aagcatattt ttttctttga aattcataag ttatcaatta tcgatggaaa      60

tgtattctat ggagaacgtt ttacccgatg aatgggtgca aaaattattt taccttcaaa     120

tctacaatca acacacgcta acttttgtga cttgatcaac tctcacctgg aaaagcaacc     180

aactacaatc aacattctat gggataatcg acaagtgagt aaaattatag ccggacctct     240

tagtacagtg tatttaaaag gggaataata ttctatcaat aggaataaaa ataaggtcag     300

cagccatgac ttttccatca ttttgaatat accttatttg tttcgggatt aattgggggt     360

cggaaatcct cttgaattca gaaacgggaa ccggaggaag gtgccggtct ttcagaaagc     420

tgtgaaaaat accaacattt ctgctgccaa gagctcaata agaagtttca aaaattgtct     480

tggatgttgc agctgtggct gctaagtaat aagacatcta ttagtatcta gatttgttag     540

accatttaac atagtgtttt aaacgatggg gttaatagat gagggttaag aagctagtta     600

tattactgtt gctgtaacgc cttcaattgt cggttacaga gcaaacatta ttgaatgtta 660

atgtaaagag tttatttgtt ttctagtaaa catatagcga ttggttagta atcactaata 720

gaaatttttc ataagtatca aaaaagtaaa cctctttttc agtctatgta ataagtaaac 780

caaggaaagg gaaaatatct acaatcaaca agccattgtt gcagcaacaa agcaactgaa 840

actacaatca acattcaata aacttgggta atttggaatt taattctctg ggacacctgt 900

ggattacaac aatcaactcg aaacttatta tacaatgtaa ataaaaattg atatgcatac 960

atgaagatca agtgaaattc catttagaat caattttttt cgaatattaa gtttcttgct 1020

ttaatttatc tgaaagtaaa tagacattcc aaattcaagt taacaaatta ataatgaatt 1080

gactagtgat ttttaagaga aaaagataag atttaaaaaa ggaaagcctt tcttgataaa 1140

tttttgaacc actttatgcc gtttcaatca taaaaacttt taagaacaca tgactggtaa 1200

aattaattta aaacaaattt aaatttccaa cgtaacattc aacaaaaatg gtgaaaacta 1260

tcacggaaat tgttaatatt aatatgtccc aaaaatagcc tttgtatgta tatgatacta 1320

atccatacat ctatggtatc tatag 1345

## Description

### Field of the invention

The present invention relates to a polycistronic gene expression cassette or construct and a transgenic insect comprising said polycistronic expression cassette or construct. Said transgenic insect is useful in biological methods for controlling pest insects. More specifically, the invention relates to a transgenic insect comprising a lethality system, wherein several coding sequences encoding for one or more lethal gene product(s) are expressed polycistronically and are separated by self-cleaving peptides. These self-cleaving peptides cause a ribosomal skipping event allowing a polycistronic translation of two or more coding sequences under the control of a single promoter element.

### Background of the invention

Pest insects can be a severe burden on agriculture by reducing crop, fruit, or vegetable yield. Additionally, pest insects can pose a threat to human and animal health by transmitting diseases. For example, the Spotted Wing Drosophila, *Drosophila suzukii,* is a severe pest for soft-skinned fruits like strawberries, raspberries, elderberries, or cherries. Its invasive potential together with worldwide impact on fruit industry has escalated its importance for both basic and applied science during the last decade. Meanwhile, the fly has spread enormously over Asia, North and South America, as well as Europe and is established in many areas. *D. suzukii* is capable of destroying whole harvests within only a couple of days under permissive environmental conditions. The short generation time of *D. suzukii,* which is typical for pest insects, together with its high fecundity is a challenge for the control of the fly by conventional pest control methods and creates a demand for new strategies. One pest control method is the sterile insect technique (SIT), which uses sterile males for population control. It works by releasing a large number of usually radiation-sterilized males into the environment, where they compete for the females with the field male population. Mating between sterilized males and the field females leads to unfertilized eggs, hence repeated release of sterile males results in the population decline (Vreysen et al., 2007). Since only sterilized males contribute to the success of SIT, a release of male-only populations makes SIT programs more efficient due to several reasons (Cáceres et al., 2002; Rendon et al., 2000): a release of both sexes will result in many inefficient mating events between sterile males and sterile females; raising both sexes increases the rearing costs, and the release of high numbers of sterilized fruit fly females would lead to additional fruit damage in the field due to egg deposition. It is therefore highly desirable to remove females from the population early during development (Franz, 2005). For some pest insects like the mosquito *Aedes aegypti,* sex separation can be done mechanically, based on a size dimorphism between male and female pupae (Gilles et al., 2014). For most species, however, this is not possible. For the medfly *Ceratitis capitata,* genetic sexing strains (GSSs) are available, which are based on a temperature sensitive lethal mechanism linked to the female sex by chromosomal translocations (Robinson et al., 1999). Such GSSs allow for efficient and early female elimination during the mass rearing process. It would be desirable to have GSSs for all pest insects where SIT can be applied. However, the generation of GSSs is time and labor intensive and needs to be established for every single species from scratch. Alternatively, it was proposed that the expression of the male determination gene (M) could be used for pest control. The expression of the male determination gene could suppress the population in disease transmitting insects for example in the genera *Aedes* or *Anopheles* (Adelman and Tu, 2016).

An alternative is the implementation of modern transgenic techniques to develop transgenic embryonic sexing strains (TESSs), which is based on sex-specific splicing and early embryonic activation of pro-apoptotic genes (Horn and Wimmer, 2003; Ogaugwu et al., 2013; Yan and Scott, 2015). The composition of sex-specificity and the killing effectors of sexing constructs can be adapted to various insect species, thus allowing the development of TESSs for many different pest insects in a reasonable timeframe. TESS also requires the production of large numbers of individuals in a mass rearing facility. Thus, the chance of mutations that render the transgenic construct non-functional is given. Those mutations can lead to resistance, which would make additional pest control measures necessary (Handler, 2016). Moreover, an unrecognized resistance during the rearing process could lead to the stable establishment of a transgenic population in the field after releases. Therefore, it is essential to make TESS as safe as possible, e.g. by creating built-in backup systems that can independently confer lethality.

The main technical issues regarding the creation of TESS are transgene expression, killing efficiency, biological safety regarding the susceptibility of the transgene system to gene mutations, and the vector size. All these subjects are interconnected. For instance, improving the robustness against gene mutations by backup systems, or expressing multiple copies of a killing gene to enhance killing efficiency would both increase the vector size, which would, in turn, reduce the cloning efficiency. Using several complete expression cassettes consisting of a promoter, a coding sequence, and a poly-A signal, would dramatically increase the size of the transgene.

2A self-cleaving peptides have been identified from the picornavirus family (Ryan et al., 1991). Examples include the 2A self-cleaving peptide sequence from the foot and mouth disease virus (FMDV) and it allows the expression of several gene products from one polycistronic mRNA. 2A peptide sequences are usually between 18 to 22 amino acids in size and have been described to act through a ribosomal skipping event, where the ribosome does not form the peptide bond between the Glycine residue at the C-terminal end of the 2A peptide and the N-terminal Proline of the downstream protein (Donnelly et al., 2001b; Ryan et al., 1999). This skipping, however, does not abolish the translation process. As a result, both polypeptides upstream and downstream of the 2A sequence are being synthesized in a ratio of approximately 1:1 (Donnelly et al., 2001a). 2A peptides have been used for equimolar expression of different gene products for biomedical research (Szymczak et al., 2004), quantitative co-expression for Foerster Resonance Energy Transfer (FRET) (Goedhart et al., 2011), and multi-gene expression systems in insects (Daniels et al., 2014; Wang et al., 2015).

There is a need in the art for an improved biological method for controlling pest insects by implementing improved lethality systems.

### Summary of Invention

The present invention relates to a polycistronic expression cassette, comprising in functional linkage two or more coding sequences encoding one or more lethal gene product(s), wherein said coding sequences encoding said lethal gene product(s) are each separated by a coding sequence encoding a self-cleaving peptide sequence, wherein the expression cassette is under the control of a single repressible promoter element, as further defined in the claims. Also, the present invention relates to an expression construct, comprising an expression cassette according to the invention and further comprising a second expression cassette, as further defined in the claims. Further, the present disclosure provides an insect cell, comprising an expression cassette according to the invention or an expression construct according to the invention, wherein the cell is a *Drosophila Schneider S2* cell, an *Anastrepha suspensa* cell, or an *Aedes aegypti* cell. Moreover, the invention relates to a transgenic insect comprising an expression cassette or an expression construct according to the invention, as also further defined in the claims. In addition, the present invention relates to the use of said transgenic insect for controlling reproduction in an insect population of interest, wherein the transgenic insect is capable of interbreeding with insects of the population of interest, preferably wherein the insects are crop pest insects or disease transmitting insects, as further defined in the claims. Finally, the present invention relates to a method of controlling reproduction in an insect population of interest, comprising the steps of (i) providing a plurality of insects according to the invention capable of interbreeding with the insects of the population of interest, (ii) optionally selecting suitable individual insects from the plurality, and (iii) allowing the insects of step (i) or (ii) to interbreed with insects of the population of interest, and optionally wherein the insects of step (i) or (ii) are released in an area where reproduction control of insects of the population of interest is desirable, and preferably wherein in step (i) the insects are propagated under conditions, wherein the promoter element is repressed; and/or in step (ii), male insects are selected from the plurality, as also described in the claims.

### Detailed description of the invention

Following the present invention, self-cleaving peptides can be advantageously used for polycistronic expression of one or more lethal gene product(s) for insect pest control.

The present invention disclosed herein makes pest control safer and more efficient. The inventors tested four different 2A peptides in two insect cell lines and generate transgenic *Drosophila suzukii* strains, showing that gene products are polycistronically expressed and successfully cleaved after translation due to efficient ribosomal skipping. The examples disclosed herein highlight the potential of these peptides for applications in insect pest control.

In particular, the present invention provides a polycistronic expression cassette, comprising in functional linkage two or more coding sequences encoding one or more lethal gene product(s), wherein said coding sequences encoding said lethal gene product(s) are each separated by a coding sequence encoding a self-cleaving peptide sequence, wherein the expression cassette is under the control of a single repressible promoter element.

In one embodiment, the expression cassette comprises up to twelve coding sequences, preferably up to eleven coding, preferably up to ten coding, preferably up to nine coding sequences, preferably up to eight coding sequences, preferably up to seven coding sequences, preferably up to six coding, more preferably up to five coding sequences, more preferably up to four coding sequences, even more preferably up to three coding sequences, such as two coding sequences encoding.

As used herein, the term "in functional linkage" refers to any order of arrangement of the elements or components of a gene expression cassette according to the invention permitting functional interactions of the elements or the component in question. For example, "in functional linkage" can mean that a set of DNA sequences are contiguously linked, or are placed in a position so as to exert regulatory effects onto corresponding genes. In the context of the invention, the term "in functional linkage" refers to the positioning of an element in the gene expression cassette according to the invention, or to the positioning of a nucleic acid, in such a way as to permit or facilitate transcription and/or translation of the nucleic acid in question.

In a "polycistronic" expression cassette, the expression of two or more proteins is made from one open reading frame, wherein coding sequences are placed in a series within the expression cassette. Thus, transcription results in a single mRNA molecule comprising two or more coding sequences.

In general, an expression cassette is composed of one or more genes and translatory and transcriptional regulatory sequences which control the expression of the gene(s). At minimum, an expression cassette usually comprises three components: a promoter sequence, an open reading frame, and a 3' untranslated region that contains a polyadenylation site in eukaryotes. According to the invention, the term "expression cassette" comprises a single promoter element and two or more coding sequences encoding one or more lethal gene products(s). Coding sequences are portions of nucleic acid sequences that directly determine the peptide sequence of the protein it is coding for. In the context of a polycistronic expression cassette, coding sequences as referred to herein do not necessarily start with a 5' start codon and end with a 3' stop codon, since two or more coding sequences can be transcribed in a single process. In such a setup, the process only requires one 5' start codon and one 3' stop codon.

A "promoter" as used herein is typically a DNA sequence located 5' to a DNA sequence to be transcribed, and is typically positioned upstream of the 5' start codon of the first exon of a coding sequence or a transcription start site. Generally, a promoter comprises a promoter element, which is a region upstream of a coding region to which RNA polymerase binds, which then drives the expression. In embodiments, the expression of said expression cassette is under the control of a promoter element, wherein the promoter element is a response element of a transactivator. Binding of a cognate transactivator to said response element induces expression of said expression cassette by promoting binding of the RNA polymerase. Induction of expression by the transactivator can be repressed in the presence of an exogenous factor.

According to the invention, the expression cassette comprises a single promoter element, which is "repressible". In the context of the present invention, it can be determined whether a promoter element is repressible by measuring the level of lethality caused by expression of the polycistronic expression cassette in a host cell under repressed and unrepressed conditions. The skilled person knows methods for measuring lethality of insect cells or insects. The expression cassette is under the control of a repressible promoter element, in case that, when cultivated under identical cell culture conditions, less than 50%, preferably less than 40%, more preferably less than 30%, even more preferably less than 20%, still more preferably less than 10%, and most preferably less than 5% of the cells die under repressed conditions as compared to a suitable control which do not express the one or more lethal gene products, and at the same time more than 95% of the cells die under non-repressed conditions as compared to a suitable control which do not express the one or more lethal gene products.

For example, a repressible promoter element can be switched-off in the presence of an exogenous factor. Specifically, the transactivator binding to the promoter element may be controllable by a suitable exogenous factor. The repressed conditions mean herein that the activity of the transactivator is repressed in the presence of an exogenous factor.

The expression of the polycistronic expression cassette is controllable by a suitable exogenous factor. For example, a repression of the activity of the transactivator can be measured by measuring the level of lethality caused by the lethal gene products or by the optional expression of a marker such as a fluorescent marker in the presence or in the absence of an exogenous factor. In embodiments, the exogenous factor is a molecule, which is active in the organism, when contacted with said organism, and which originated outside said organism. In an alternative embodiment, the exogenous factor may be a physic-chemical factor, such as a particular temperature, etc.

The expression of said transactivator is under the control of a promoter, wherein said promoter may be a developmental-stage specific promoter or a constitutive promoter. In case the expression of said transactivator is under the control of a developmental stage specific promoter, the expression of the lethal gene product(s) is regulated with a developmental-stage specific promoter element, as further defined in the present disclosure below. The term "constitutive promoter" as used herein refers to a promoter, which constitutively drives expression of the transactivator. As a consequence, expression occurs in different tissues and different developmental stages. In a preferred embodiment, said transactivator is under the control of a developmental stage specific promoter.

In a preferred embodiment, the repressible promoter element is a tetracycline repressible transactivator (tTA) response element, which forms part of the Tet-Off system. In a Tet-Off system, expression of response element-controlled genes can be repressed by tetracycline and its derivatives. They bind tTA (or a functional derivative thereof) and render it incapable of binding to its response element sequences, thereby preventing transactivation of response element-controlled genes; in the context of the present invention of the polycistronic expression cassette. In this constellation, the suitable exogenous factor is tetracycline, or a functional derivative thereof. Examples for functional derivatives of tetracycline include, but are not limited to doxycycline, 4-epidoxycycline, anhydrotetracycline, 4-epi-oxytetracycline, chlorotetracycline, and cyanotetracycline. A skilled person can determine suitable amounts of exogenous factor for use in accordance with the invention. Typically, if the Tet-Off systems used, tetracycline is supplied in concentrations ranging between 1 and 100 µg/ml.

Further examples for suitable transactivator in systems are known in the art and include e.g. the GAL4-ER system, which is based on steroid hormone responsive transcription factors or the classical EAL4-UAS system (TARGET), which is based on the GAL4 transcriptional activator from yeast as a first component, and UAS binding sites together with a temperature-sensitive allele of the GAL80 factor as a second component. Suitable exogenous factors for repressing the expression cassette are in such a context, e.g., steroid hormones or physical effects such as applying a heat shock.

The term "lethal gene product" refers to proteins inducing death in an insect or insect cell, if expressed in said insect or insect cell. In the context of the present invention, lethality is induced due to the expression of two or more coding sequences encoding one or more lethal gene product(s). In certain embodiments, lethal gene product(s) can be pro-apoptotic or apoptotic gene product(s).

The term "pro-apoptotic" refers to gene products promoting programmed cell death, also called apoptosis, and thereby rendering an insect cell or a whole insect susceptible to apoptosis, thereby promoting apoptosis. In contrast, the term "apoptotic" genes refers to gene products, which are directly involved in the apoptotic process. One type of apoptotic genes are "caspases", which are cysteine-aspartic proteases. Caspases initiate or execute apoptosis and fulfill their role by cleaving a target protein after an aspartic acid residue. Preferred pro-apoptotic genes are *head involution defective (hid), grim, michelob_x* (*mx*), *reaper* (*rpr*), *sickle* (*skl*), *NippleDm,* orthologs thereof, or functional derivatives thereof. In a more preferred embodiment, the pro-apoptotic gene is *hid,* an ortholog thereof, or functional derivatives thereof. An example of a functional derivative of *hid* is *hid*^{Ala5}*.* Exemplary apoptotic gene(s) are genes encoding caspases. In one embodiment, the apoptotic gene encodes the caspase *dronc* (Death regulator Nedd2-like caspase), an ortholog thereof, or a functional derivative thereof.

According to the invention, pro-apoptotic and apoptotic gene products are isolated from the order Diptera preferably from the families *Tephritidae* or *Culicidae,* preferably *Ceratitis capitata, Aedes aegypti, Drosophila melanogaster, Anastrepha ludens, Anastrepha suspensa, Anopheles gambiae,* or *Drosophila suzukii,* most preferably *Ceratitis capitata, Aedes aegypti,* or *Drosophila melanogaster.* For example, the pro-apoptotic gene fragments of *head involution defective* (*hid*) and *reaper* (*rpf*) from *Anastrepha suspensa* are 732 and 380 bp in size, respectively (Schetelig et al., 2011; incorporated herewith by reference) and therefore polycistronic expression cassettes can be cloned and transfected into an insect efficiently.

An "ortholog" refers to homologous gene sequences found in different species. Orthologs also refer to fragments of a lethal gene product, which directly result in a lethal effect or promote lethality. Functional derivatives refer to gene sequences, which are fulfilling the same biological function. The coding sequences of pro-apoptotic and apoptotic genes listed above can be isolated from any species listed above and coding sequences from different species can be combined within an expression cassette.

In one embodiment, at least one of said coding sequences encoding said one or more lethal gene product(s) encodes a Ds-Red-CAAX fusion protein, preferably wherein in said DsRed-CAAX fusion protein a poly-lysine-CAAX membrane tag is fused C-terminally to DsRed. DsRed is a fluorescent protein originally isolated from *Discosoma.* According to the invention, CAAX membrane binding motif contains a lysine rich poly basic sequence followed by a C-terminal CAAX motif, wherein "C" is a cysteine, "A" is an aliphatic amino acid, and "X" is any amino acid. DsRed-CAAX can be implemented as a substitute for a lethal gene product in the expression cassette, with the advantage of being usable in all insect species of interest. A fusion protein of DsRed and CAAX is a single amino acid chain, which is not separated during the process of translation, in particular not separated by a self-cleaving peptide sequence. In particular embodiments, the poly-lysine-CAAX membrane tag has the amino acid sequence shown in SEQ ID NO. 2.

As demonstrated in Example 3, 5, and 6 of the present disclosure, the CAAX tag efficiently localized proteins to the cell membrane, and a lethal effect for membrane-bound DsRed (DsRed-CAAX) is exemplified in Example 6. The membrane-bound DsRed protein formed cluster agglomeration with detrimental effects on the cell survival in cell culture and larvae tested (Example 6). To our knowledge, these effects have not been described in the art and the DsRed-CAAX protein can serve as a novel, universal effector tool for insect control programs. In addition, expression of DsRed-CAAX is an easy to screen internal marker, which facilitates quality controls to search for potential loss of function mutations during mass rearing. DsRed is advantageous, because it is a widely used marker in molecular biology, has been used in plants, vertebrates, and invertebrates, and is not expected to be harmful to humans (Pavely and Fedorova, 2006; Vintersten et al., 2004; each incorporated herewith by reference). So far no environmental risks due to the use of DsRed could be found, a study assessing the potential risk of DsRed marked mosquitoes for predators of the *Toxorhynchites* genus, could not detect any negative impact on the predators after feeding *Aedes aegypti* larvae carrying a DsRed transgene (Nordin et al., 2013; incorporated herewith by reference). Hence, this universal effector also helps in minimizing the effort to test the transgene for potential detrimental effects on human health or the environment.

In the polycistronic expression cassette of the present invention, two or more coding sequences encoding gene product(s) are controlled by one promoter element, thereby reducing the size of the expression cassette. The reduced size provides for better cloning, transformation, and transfection efficiency. Moreover, the polycistronic expression of gene products is advantageous compared to the use of several expression cassettes, because in the latter an individual promoter is required for each gene product. Individual expression cassettes can lead to unequal expression strength due to different promoter strengths or varying genomic position effects. Moreover, the repeated use of the same promoter sequence in one expression cassette can have adverse effects on coding sequence stability - an effect that can be overcome by the polycistronic expression cassette presented herein. Polycistronic expression of several coding sequences also bears the advantage of having a higher resistance against point/missense mutations. A polycistronic expression cassette can cope with the loss of one coding sequence due to a point/missense mutation because there would still be one or more functional coding sequences on the same expression cassette. Accordingly, using a polycistronic expression cassette according the present invention in pest control makes elimination of pest insects safer and more efficient.

The expression cassette can be cloned by standard molecular biology techniques making use of either traditional restriction enzyme cloning or modern recombination cloning techniques like Gibson cloning (New England Biolabs) or gene synthesis.

In the context of the present invention, the two or more coding sequences encode one or more lethal gene product(s). In one embodiment, said two or more coding sequences encode one lethal gene product. Within this embodiment, the polycistronic expression cassette may express several copies of the same lethal gene. In case the polycistronic expression cassette expresses several copies of the same lethal gene, the advantage of the polycistronic expression cassette of the present invention is a faster, higher killing efficiency as compared to expression cassettes expressing just a single coding sequence of a lethal gene product. This embodiment may be of particular interest if the lethal gene product is an apoptotic gene.

In another embodiment, said two or more coding sequences encode two or more lethal gene products. This is of particular advantage if the lethal gene products comprise two or more pro-apoptotic genes, since in this case the expression of several different lethal genes under the control of a single promoter is expected to result in a synergistic effect of the pro-apoptotic genes. The synergistic effect of two lethal gene products expressed from different expression cassettes has been demonstrated shown for pro-apoptotic genes *hid* and *reaper* used in sex-specific killing systems (Schetelig et al., 2011; Zhou et al., 1997; each incorporated herewith by reference).

The polycistronic expression results in the translation of two or more lethal gene products in stoichiometric amounts in a ratio in the range of 0.9:1 to 1.1:1. Stoichiometry is a mechanistic and inherent feature of the system as polycistronic expression is achieved by ribosomal skipping during the process of translation because ribosomal skipping does not abolish the translation process. Stoichiometry could also be detected by mass spectrometry approaches. The stoichiometric expression of the individual gene products offers the opportunity to have an equal expression of the synergistic genes in the necessary 1:1 ratio. It has been shown that stoichiometric separation efficiency can be reached with 2A peptides (Daniels et al., 2014; Donnelly et al., 2001a; each incorporated herewith by reference). Accordingly, in one embodiment the polycistronic expression cassette comprises two or more coding sequences encoding *hid* and *reaper.* Also for this reason expressing several copies of the same lethal genes or a combination of different lethal genes makes pest control safer and more efficient.

According to the invention, coding sequences encoding self-cleaving peptide sequences are DNA sequences, which can be placed between two coding sequences. After transcription of the expression cassette, the resulting mRNA molecule also contains coding sequences coding for said self-cleaving peptide sequence(s). The process of translation of said coding sequences coding for said self-cleaving peptide sequence(s) results in ribosomal skipping. Ribosomal skipping is an event, wherein a peptide bond between two amino acids is not formed, so that the already translated amino acid chain is released from the ribosome. However, the process of translation is not aborted, but proceeds further. As a result, separate protein products are released from the ribosome.

In one embodiment, the self-cleaving peptides are 2A viral peptide sequences. 2A viral peptide sequences can, for example, be isolated from the picornavirus family. Preferred 2A viral peptide sequences are selected from the group consisting of (i) 2A from *Thosea asigna* virus as shown in SEQ ID NO. 3, (ii) 2A from *Drosophila* C virus as shown in SEQ ID NO. 4, (iii) 2A from Foot and mouth disease virus (2A1/31) as shown in SEQ ID NO. 5, and (iv) 2A from Foot and mouth disease virus (2A1/32) as shown in SEQ ID NO. 6. The data outlined in the examples herewith confirm the high efficiency of 2A peptide-mediated 'cleavage' in insect cell culture and whole insects. Examples 2, 3, and 5 exemplify the functionality of the 2A peptide sequence mediated cleavage system in insect cells and in whole insects. The 2A peptide expression analysis *in vivo* in *D. suzukii* illustrates the different applications in insects and shows applicability of 2A polycistronic cleavable peptides for disease transmitting insects and for crop pest insects. 2A peptides are usually between 18 and 22 amino acids in size and therefore have little influence on overall expression cassette size. This is a significant advantage over multi-gene expression for example requiring several promoter sequences or IRES sequences, which are often over 500bp in size. Multi-gene expression cassettes easily add up to an insert size of 10 kilobases or more, which is detrimental for bacterial transformation efficiency and in the procedure of transposon- or recombination technology-mediated transfection of insects.

The use of self-cleaving peptides has the further advantage that several individual protein products can be translated in a single process, which makes expression more efficient, as translation initiation is believed to be the rate limiting step during translation (Shah et al., 2013; incorporated herewith by reference). An alternative method for polycistronic expression applies internal ribosomal entry sites (IRES) on the mRNA level. However, IRES sequences often show uneven expression of the gene products upstream and downstream of the IRES sequence, with the gene upstream being usually stronger expressed than the downstream gene (Trichas et al., 2008; incorporated herewith by reference). In contrast thereto, polycistronic expression using self-cleaving peptide sequences shows stoichiometric expression of the gene products. Therefore, the polycistronic expression cassette disclosed herein is an improved expression cassette providing equal expression of coding sequences as compared to polycistronic expression using internal ribosomal entry sites.

In one embodiment, the expression of lethal gene product(s) is regulated with a developmental-stage specific promoter element and/or a sex-specific intron.

The term "developmental stage" as used herein refers to a stage during development of an insect. Thus, the expression cassette under the control of a developmental stage specific promoter is activated during development of a transgenic insect via expression of the transactivator, and causes lethality, preferably at an embryonic stage of development. In further scenarios, lethality would occur in larval, pupal or adult stages. However, embryonic stages during development are preferred.

Accordingly, regulating the expression of one or more lethal gene products using a "developmental stage specific promoter element" as used herein refers to a promoter that is active or capable of being activated in an insect during a certain stage during development or adult life of said insect. Activation of the promoter element results in expression of a repressible transactivator (as further described above), which in turn activates expression of the polycistronic expression cassette. Accordingly, when the expression of the expression cassette is regulated by a "developmental stage specific promoter element", the expression cassette is expressed at a specific developmental stage of an insect and thereby causes lethality of the insect during said developmental stage. The expression cassette according to the invention can be suitably expressed in an insect preferably during an embryonic developmental stage. This has various advantages: Firstly, released insects mate with wild-type insects, which offers the advantage of inhibiting larval development in the field, which ensures crop, fruit, vegetable, and the like quality and quantity and reduces the quantity of disease transmitting insects. Secondly, developmental stage specific promoter, particularly the embryonic stage specific promoter ensures that the lethal expression cassette is exclusively expressed in a particular developmental stage, preferably exclusively in embryos. Other promoter elements, which are active throughout development only in later stages, might cause side effects leading to decreased fitness of the strains and a lower efficiency during field release.

Therefore, in a preferred embodiment, said promoter is a developmental stage specific promoter, more preferably wherein the developmental-stage specific promoter is an embryonic stage specific promoter, most preferably a promoter which is activated in the embryonic stage of cellularization. Typical characteristics of the cellularization stage are known to the persons skilled in the art. The process of cellularization involves integrating mechanisms of cell polarity, cell-cell adhesion and a specialized from of cytokinesis, which ends up in a monolayer of blastoderm cells.

In addition, or in the alternative, regulating the expression of one or more lethal gene products using a "sex-specific intron" refers to an intron, which is spliced out either in females or males specifically due to alternative splicing. When the intron is completely spliced out, the lethal gene product(s) is/are expressed. If the intron is not completely spliced out, the expression cassette is not translated fully due to a premature stop codon, or the expression cassette is not translated in sense with regard to the lethal gene product(s). When the expression of the expression cassette is regulated by a "sex-specific intron", the expression cassette is either expressed in males or females and thereby causes lethality in either males or females.

In one embodiment, the sex-specific intron herein is positioned (i) at any position between the transcription start site and the first coding sequence of a lethal gene product, or (ii) within the first coding sequence of a lethal gene product. It is preferred herein that the sex-specific intron is placed (i) at any position between the translation start site and the first coding sequence of a lethal gene product, or (ii) within the first coding sequence of a lethal gene product. Preferably, the sex-specific intron is a female-specific intron. More preferably the sex-specific intron is a female-specific intron. Most preferably, the *Cctransformer-I* intron with the sequence shown in SEQ ID NO. 30 is used.

In a further embodiment, the expression cassette can further comprise in functional linkage a coding sequence encoding a marker gene, wherein said marker gene is separated by a self-cleaving peptide. As a result, the marker gene is expressed in stoichiometric amounts with the lethal gene product(s), when the polycistronic expression cassette is translated. The marker gene allows the detection of expression in an insect cell or in an insect. A variety of suitable marker genes are known in the art offering expression detection by means such as optical or immunological methods. Preferably herein, the marker genes used in the context of the invention allow optical expression detection, such as by way of fluorescent marker protein. A multitude of suitable fluorescent protein of different colors and other properties are known in the art. Examples include AmCyan, green fluorescent protein (GFP), DsRed, enhanced green fluorescent protein (EGFP), blue fluorescent protein (BFP), red fluorescent protein (RFP), yellow fluorescent protein (YFP). The term AmCyan describes a protein derived from am FP486, which was originally cloned from the sea anemone *Anemonia majano* (GenBank accession number AF168421). AmCyan belongs to the family of fluorescent proteins isolated from coral reef organisms, similarly to green fluorescent protein (GFP), which was originally derived from the jellyfish. Preferred fluorescent proteins according to the invention are AmCyan, DsRed, or EGFP. The most preferred marker genes according to the invention are fluorescent proteins such as DsRed or EGFP. As noted above, the stoichiometric expression of the individual gene products offers the opportunity to have an equal expression of the genes in a 1:1 ratio, which allows monitoring of the effector protein expression as a quality control.

In another embodiment, one or more of said coding sequences encode(s) a fusion protein comprising a C-terminal nuclear localization sequence. A nuclear localization sequence fused to protein has the advantage that a protein of interest is settled efficiently into the nucleus of a cell to for example exert an action within the nucleus. A preferred nuclear localization sequence herein has the amino acid sequence shown in SEQ ID NO. 1 from the *D. suzukii* transformer gene. As shown in the examples 3, 5, and 6, the nuclear localization sequence directed the tagged proteins to the cell nucleus and can be used for subcellular localization of other proteins. The nuclear localization sequences are particularly advantageous when fused to proteins, which are enzymes with a function within the nucleus, such as Cas9 proteins, transposases and recombinases. In this manner, the nuclear localization sequences help shuttling potential killing effector genes in early embryonic lethality to the nucleus. Further provided is an expression construct, which comprises a first polycistronic expression cassette as described above, and which further comprises a second expression cassette. The second expression cassette comprises one coding sequence, or, in functional linkage, two or more polycistronically expressed coding sequences, wherein the two or more polycistronically expressed coding sequence are each separated by a coding sequence encoding a self-cleaving peptide sequence, wherein said encoded self-cleaving peptide sequence is a 2A viral peptide sequence. The 2A viral peptide sequence may be one as described herein above for the first polycistronic expression cassette.

In an embodiment, the second expression cassette encodes one or more sex-determining gene(s). Preferably, these genes are one or more male-determining gene(s) (M-factor), most preferably the M-factor on the Y-chromosome of *Ceratitis capitata, the nix* gene from *Aedes aegypti,* the *GUY1* gene from *Anopheles stephensi,* or the *mdmd* gene from *Musca domestica.* The expression of one or more sex determination genes promotes the development of male-only strains.

The term sex-determining gene(s) used herein refers to genes, which promote the development of males or females in insects. Generally, the sex of an insect is naturally regulated by the ratio of the number of X-chromosomes to the number of autosomes. The balance between female-determining factors encoded on the X-chromosome and male-determining factors encoded on the autosomes determines the sex of an insect. Depending on the ratio of chromosomes, a sex-specific pattern of transcription will be initiated and the sex of an insect is determined. In addition, some insects additionally encode a male determining factor on the Y-chromosome (Adelman and Tu, 2016; incorporated herewith by reference).

In an embodiment, the second expression cassette comprises a fluorescent marker. Possible fluorescent markers are those as described herein in the context of the first polycistronic expression cassette

In a further embodiment, the second expression cassette additionally comprises a sex-specific intron positioned, as defined above, at (i) any position between the transcription start site, preferable the translation start site, and the first coding sequence of said second expression cassette, or (ii) within the first coding sequence of said second expression cassette, preferably wherein said sex-specific intron is a female-specific intron. More preferably, the second expression cassette comprises the sex-specific intron of the sex determination gene transformer (*Cctransformer-I* intron), preferably the *Cctransformer-I* intron having the sequence shown in SEQ ID NO. 30.

Moreover, in further embodiments, the expression of said second expression cassette is under the control of a promoter element, wherein the promoter element is a response element of a transactivator. Binding of said transactivator to said response element induces expression of said second expression cassette. The transactivator of the second expression cassette can be under the control of a developmental stage-specific promoter or a constitutive promoter, each as described above with regard to the first polycistronic expression cassette. Furthermore, the expression of said second cassette can be repressed in the presence of a repressor acting on said transactivator. Similarly to the transactivator, the repressor can also be under the control of a developmental stage specific promoter or a constitutive promoter, wherein the developmental stage specific promoter or the constitutive promoter may be one as described above with regard to the first polycistronic expression cassette. In addition, the repressor can be repressed in the presence of an exogenous factor. When a repressor and transactivator is present, the addition of an exogenous factor leads to an increase of expression of the second expression cassette, because the repressor is repressed.

In a preferred embodiment, the transactivator of the second expression cassette is QA-1F isolated from *Neurospora crassa,* the repressor is QA-1S from *Neurospora crassa,* the response element comprises QA-1F binding sites, and the exogenous factor is quinic acid.

In addition, the present disclosure also provides an insect cell, which comprises a polycistronic expression cassette or an expression construct according to the present disclosure. Preferred insect cells include a *Drosophila Schneider S2* cell, an *Anastrepha suspensa cell,* or an *Aedes aegypti* cell.

Moreover, the present disclosure further provides an animal belonging to the class insecta, which comprises a polycistronic expression cassette or an expression construct according to the present disclosure. An insect comprising a polycistronic expression cassette or an expression construct qualifies as a "transgenic insect".

"Transgenic insects" according to the invention belong preferably to the order *Diptera,* and are more preferably crop pest flies or disease transmitting flies belonging to the families *Tephritidae* or *Culicidae.* Even more preferred genera are *Drosophilidae, Anastrepha,* or *Aedes.* Most preferred species belong to the group *D. suzukii, D. melanogaster, Anastrepha suspensa,* or *Aedes aegypti,* in particular the insect species *D. suzukii.*

Generally, the term "insect pest species" includes injurious or unwanted insects and insects recognized as a destroyer of economic goods or a risk for animal and human health. Insect pest species can be subdivided into "crop pest species" and "disease transmitting species". Crop pest species, also referred to as "agricultural pest species", for example, inflict damage on agricultural products such as crops, fruit, vegetables, and the like, and are therefore of economical relevance. Insects often become crop pest species when the ecological balance is interrupted by human intervention or natural events, which leads to an overgrowth of these species. Crop pest species can for example reduce or even destroy whole harvests of crops, in case of *Drosophila suzukii,* soft-skinned fruits like strawberries, raspberries, elderberries, or cherries.

The term "disease transmitting insects" refers to insects, which are able to transmit diseases between humans and animals. Thereby, the insects can carry pathogens, which include viruses, bacteria, or parasites. Examples of pathogens are viruses such as dengue fever or yellow fever, parasites such as *Plasmodium falciparum* causing malaria, or bacteria such as *Borrelia burgdorferi* causing Lyme disease. However, it is also contemplated that the polycistronic expression cassette of the invention can also be used in insect species that are not pest species.

In one embodiment, the expression cassette or construct is integrated into the insect by transfecting the insect with the expression cassette or construct, thereby resulting in the transgenic insect. The expression cassette or constructs is stably integrated into the genome of the transgenic insect. Stable integration can either occur via a random stable process or a targeted stable process. During a random stable process integration locus of the expression cassette or construct is not predetermined. Multiple integration events are also possible. During a targeted stable process, the locus of integration is preferred by homologous sequences between the expression cassette or construct and the genomic locus. Due to a targeted integration, the expression level of the expression cassette or construct is expected to be similar in all transgenic insects produced during the rearing process. The reduced size of the expression cassette or construct due to a polycistronic expression only requiring a single promoter element is advantageous for genomic integration because genome integration efficiency decreases with increasing expression cassette or construct size. Random stable integration can be achieved by the use of a transposon system. For the insect-derived transposons like *Minos, Hermes, Mosl,* and *piggyBac,* the optimal range of the cargo size is ≤10 kb (Atkinson et al., 2001; incorporated herewith by reference).

In a preferred embodiment, the *piggyBac* system is used for random stable integration and integration is possible at any TTAA chromosomal sites. Other options for random stable integration are the *Minos, Hermes,* or *Mos1* transposases and the person skilled in the art knows how to use these systems.

In another preferred embodiment "Recombinase-Mediated Cassette Exchange" (RMCE) or a "CRISPR/Cas-based system" are used for targeted stable integration. Most preferred herein is the use of the *piggyBac* system for random stable integration. The *piggyBac* system is a transposon system, which is used to introduce exogenous DNA, in this case the expression cassette or construct, into the insect genome.

In one embodiment, the transgenic insect according to the invention comprises two copies of an expression cassette or two copies of an expression construct. The presence of two copies of the expression cassette or construct makes the system more reliable because the second cassette or construct can serve as a backup in case the other cassette or construct suffered from a mutation leading to a non-lethal expression of gene product(s).

Further provided is a use of a transgenic insect according to the present disclosure for controlling reproduction in an insect population of interest, wherein the transgenic insect is capable of interbreeding with insects of the population of interest as defined herein.

The term "controlling reproduction" of an insect population as used herein includes a directed influence on the number of offspring produced in any given insect population in a defined area. Preferably, reproduction control according to the uses and methods of the invention results in a decrease of the number of offspring of an insect population of interest, preferably in a developmental-stage specific manner. Preferably, controlling reproduction eventually results in the elimination, suppression, containment, or prevention of an insect population of interest or parts thereof in a defined area, and exclude a new introduction of such insects from other areas into the area of interest. For example, an eradication program has the ability to eliminate complete pest populations in a species-specific manner and leads to a reduction in the use of insecticides, leading to a long-term benefit for the environment.

"An insect population of interest" as used herein means a number of insects of a particular species, typically living in a defined area such as contained in a laboratory or a rearing facility, or living in a given geographic area. Insect populations of interest are the targets of the lethality system of the invention. Of particular interest according to the invention are insect populations that act as pests in natural habitats, cultivated land, or the anthroposphere. This includes crop pest insects and disease transmitting insects as defined herein.

Furthermore, the present disclosure also provides a method of controlling reproduction in an insect population of interest, comprising the steps of (i) providing a plurality of insects according to the invention capable of interbreeding with the insects of the population of interest, (ii) optionally selecting suitable individual insects from the plurality, and (iii) allowing the insects of step (i) or (ii) to interbreed with insects of the population of interest. Preferably, the insects of step (i) or (ii) are released in an area where reproduction control of insects of the population of interest is desirable. The reproduction control is part and parcel of environmental-friendly area-wide insect pest management programs.

The meaning of the terms "controlling reproduction" and "insect population of interest" has been further explained above.

"Providing a plurality of insects" as used herein means the provision of insects according to the present disclosure in numbers and quality, sufficient for the intended purpose of controlling reproduction in an insect population of interest. Method for producing transgenic insects comprising the stable integration of the lethality inducing an expression cassette or expression construct are provided in the present disclosure above. In certain embodiments, the insects are propagated under conditions, wherein the expression of the lethal gene product(s) is repressed. Further methods of providing a plurality of insects by techniques such as breeding, rearing insects, and evaluating their suitability for use in the methods of the invention are known in the art.

Finally, the present disclosure provides a process of generating a transgenic insect comprising the step of introducing an expression cassette or construct according to the present disclosure into an insect. Methods for introducing the expression cassette or construct of the present disclosure are provided above in the context of the transgenic insect of the present disclosure.

In one embodiment, the expression cassette or construct is introduced into the insect by random stable integration into the germline genome or by targeted stable integration into the genome, wherein preferably the *piggyBac* system is used for random stable integration, and wherein preferably Recombinase-Mediated Cassette Exchange or a CRISPR/Cas-based system is used for targeted stable integration, as described above.

In another embodiment, the process further comprises the step of screening hatched G₀ larvae for expression of the expression cassette or the expression construct, preferably wherein the hatched G₀ larvae can be screened for the presence of a fluorescent marker.

In a preferred embodiment, the process further comprises the step of establishing independent homozygous strains by single pair inbreeding for successive generations, preferably wherein interbreeding is carried out by backcrossing of G₀ adults with WT males or virgin females.

The following a further preferred embodiments of the present disclosure:
1) A polycistronic expression cassette, comprising in functional linkage two or more coding sequences encoding one or more lethal gene product(s), wherein said coding sequences encoding said lethal gene product(s) are each separated by a coding sequence encoding a self-cleaving peptide sequence, wherein the expression cassette is under the control of a single repressible promoter element.
2) The expression cassette of embodiment 1, wherein the expression of said lethal gene product(s) is regulated with a developmental-stage specific promoter element and/or a sex-specific intron.
3) The expression cassette of embodiment 1 or 2, wherein expression of the polycistronic expression cassette results in the translation of two or more lethal gene product(s) in stoichiometric amounts in a ratio in the range of 0.9:1 to 1.1:1.
4) The expression cassette of any one of embodiments 1-3, wherein the expression cassette comprises up to twelve coding sequences encoding one or more lethal gene product(s), preferably up to eleven coding sequences, preferably up to ten coding, preferably up to nine coding sequences, preferably up to eight coding sequences, preferably up to seven coding sequences, preferably up to six coding sequences encoding for one or more lethal gene product(s), more preferably up to five coding sequences encoding one or more lethal gene product(s), more preferably up to four coding sequences encoding one or more lethal gene product(s), even more preferably up to three coding sequences encoding one or more lethal gene product(s), such as two coding sequences encoding one or more lethal gene product(s).
5) The expression cassette of any one of embodiments 1-4, wherein said two or more coding sequences encoding said one or more lethal gene product(s) are copies of the same lethal gene.
6) The expression cassette of any one of embodiments 1-5, wherein said two or more coding sequences encoding said one or more lethal gene product(s) are copies of different lethal genes.
7) The expression cassette of any one of embodiments 1-6, wherein at least one of said coding sequences encoding said one or more lethal gene product(s) are one or more pro-apoptotic gene(s) or apoptotic gene(s).
8) The expression cassette of embodiment 7, wherein said one or more pro-apoptotic gene(s) encoding *head involution defective (hid), grim, michelob_x (mx), reaper (rpr), sickle (skl), NippleDm,* an ortholog thereof, or a functional derivative thereof; preferably *hid,* an ortholog thereof, or functional derivatives thereof, in particular wherein said functional derivative is *hid*^{Ala5}; or wherein said one or more apoptotic gene(s) encoding one or more caspase(s), preferably *drone,* an ortholog thereof, or a functional derivative thereof.
9) The expression cassette of embodiment 8, wherein one or more pro-apoptotic or apoptotic gene(s) is/are isolated from Dipterans, preferably from the families *Tephritidae* or *Culicidae,* preferably *Ceratitis capitata, Aedes aegypti, Drosophila melanogaster, Anastrepha ludens, Anastrepha suspensa, Anopheles gambiae,* or *Drosophila suzukii,* most preferably *Ceratitis capitata, Aedes aegypti,* or *Drosophila melanogaster.*
10) The expression cassette of any one of embodiments 1-9, wherein at least one of said coding sequences encoding said one or more lethal gene product(s) encodes a DsRed-CAAX fusion protein, preferably wherein in said DsRed-CAAX fusion protein a poly-lysine-CAAX membrane tag is fused C-terminally to DsRed, in particular wherein said poly-lysine-CAAX membrane tag has the amino acid sequence shown in SEQ ID NO. 2.
11) The expression cassette of any one of embodiments 1-10, wherein said encoded self-cleaving peptide sequence is a 2A viral peptide sequence.
12) The expression cassette of embodiment 11, wherein said 2A viral peptide sequence is selected from 2A viral peptide sequences of the picornavirus family, preferably wherein said 2A viral peptide sequence is selected from the group consisting of (i) 2A from *Thosea asigna* virus as shown in SEQ ID NO. 3, (ii) 2A from *Drosophila* C virus as shown in SEQ ID NO. 4, (iii) 2A from Foot and mouth disease virus (2A1/31) as shown in SEQ ID NO. 5, and (iv) 2A from Foot and mouth disease virus (2A1/32) as shown in SEQ ID NO. 6.
13) The expression cassette of any one of embodiments 1-12, wherein expression by the repressible promoter element is inducible by a transactivator, and wherein said transactivator is under the control of a promoter, wherein said promoter is a developmental-stage specific promoter or a constitutive promoter, preferably wherein said promoter is a developmental stage specific promoter, more preferably wherein the developmental-stage specific promoter is an embryonic stage specific promoter, most preferably wherein the promoter is the cellularization specific promoter; and wherein induction of expression by the transactivator is repressed in the presence of an exogenous factor.
14) The expression cassette of any one of embodiments 1-12, wherein the repressible promoter element comprises a response element of a transactivator, wherein binding of said transactivator to said response element induces expression of said polycistronic expression cassette, and wherein the expression of said transactivator is under the control of a promoter, wherein said promoter is a developmental-stage specific promoter or a constitutive promoter; preferably wherein said promoter is a developmental stage specific promoter, more preferably wherein the developmental-stage specific promoter is an embryonic stage specific promoter, most preferably, wherein the promoter is a cellularization specific promoter, wherein the binding of said transactivator to said response element is repressed in the presence of an exogenous factor.
15) The expression cassette of embodiments 13 and 14, wherein the exogenous factor is tetracycline or a derivative thereof, preferably wherein the derivative is selected from the group consisting of doxycycline, 4-epidoxycycline, anhydrotetracycline, 4-epi-oxytetracycline, chlorotetracycline, and cyanotetracycline.
16) The expression cassette of any one of embodiments 1-15, comprising in functional linkage a marker gene, wherein said marker gene is separated by a coding sequence encoding a self-cleaving peptide sequence.
17) The expression cassette of embodiment 16, wherein said marker gene(s) is/are encoding (a) fluorescent marker protein(s), preferably wherein said marker protein(s) is/are DsRed or EGFP.
18) The expression cassette of any one of embodiments 1-17, wherein at least one of said coding sequences encoding said one or more lethal gene product(s) encodes a fusion protein comprising a C-terminal nuclear localization sequence, in particular wherein said nuclear localization sequence has the amino acid sequence shown in SEQ ID NO. 1 from the *D. suzukii* transformer gene.
19) The expression cassette of any one of embodiments 1-18, wherein the expression of lethal gene product(s) is regulated in a sex-specific manner, and wherein said expression cassette additionally comprises a sex-specific intron positioned at (i) any position between the transcription start site, preferably the translation start site, and the first coding sequence of a lethal gene product, or (ii) within the first coding sequence of a lethal gene product, preferably wherein said sex-specific intron is a female-specific intron.
20) The expression cassette of embodiment 19, wherein the sex-specific intron is the intron of the sex determination gene transformer *(Cctransformer-I* intron), preferably the Cctransformer-I intron having the sequence shown in SEQ ID NO. 30.
21) An expression construct, comprising an expression cassette according to any one of embodiments 1-20, and further comprising a second expression cassette.
22) The expression construct of embodiment 21, wherein said second expression cassette comprises one coding sequence or in functional linkage two or more coding sequences expressed polycistronically, wherein the two or more coding sequence are each separated by a coding sequence encoding a self-cleaving peptide sequence, wherein said encoded self-cleaving peptide sequence is a 2A viral peptide sequence.
23) The expression construct of embodiment 21 or 22, wherein said second expression cassette comprises a fluorescent marker.
24) The expression construct of any one of embodiments 20-23, wherein said second expression cassette encodes one or more sex-determining gene(s), preferably a male-determining gene (M-factor), most preferably the M-factor on the Y-chromosome of *Ceratitis capitata, the nix* gene from *Aedes aegypti,* the *GUY1* gene from *Anopheles stephensi,* or the *mdmd* gene from *Musca domestica.*
25) The expression construct of any one of embodiments 20-24, wherein the second expression cassette comprises a sex-specific intron positioned at (i) any position between the transcription start site, preferable the translation start site, and the first coding sequence of said second expression cassette, or (ii) within the first coding sequence of said second expression cassette, preferably wherein said sex-specific intron is a female-specific intron.
26) The expression construct of embodiment 25, wherein the sex-specific intron within the second expression cassette is the intron of the sex determination gene transformer (*Cctransformer-I* intron), preferably the Cctransformer-I intron having the sequence shown in SEQ ID NO. 30.
27) The expression construct of any one of embodiments 20-26, wherein induction of expression of said second expression cassette is under the control of a promoter element, wherein the promoter element is a response element of a transactivator.
28) The expression construct of any one of embodiments 20-27, wherein the transactivator is either under the control of a developmental-stage specific promoter, or wherein the transactivator is under the control of a constitutively expressed promoter; and wherein binding of said transactivator to said response element induces expression of said second expression cassette.
29) The expression construct of embodiment 27 or 28, wherein the expression of said second cassette is repressed in the presence of a repressor acting on said transactivator, wherein the repressor is under the control of a developmental stage specific promoter or a constitutively expressed promoter, and wherein the repressor is repressed in the presence of an exogenous factor.
30) The expression construct of any one of embodiments 27-29, wherein the transactivator is QA-1F isolated from *Neurospora crassa,* the repressor is QA-1S from *Neurospora crassa,* the response element comprises QA-1F binding sites, and wherein the exogenous factor is quinic acid.
31) An insect cell, comprising an expression cassette according to any one of embodiments 1-20, or an expression construct according to any one of embodiments 21-30, preferably wherein the cell is a *Drosophila Schneider S2* cell, an *Anastrepha suspensa cell,* or an *Aedes aegypti* cell.
32) A transgenic insect comprising an expression cassette according to any one of embodiments 1-20, or an expression construct according to any one of embodiments 21-30.
33) The transgenic insect as defined in embodiment 31, wherein the insect belongs to Dipterans, preferably crop pest flies or disease transmitting flies belonging to the families *Tephritidae* or *Culicidae,* preferably to the genus *Drosophilidae, Anastrepha,* or *Aedes,* more preferably of the group *D. suzukii, D. melanogaster, Anastrepha suspensa,* or *Aedes aegypti,* most preferably wherein the insect is a *D. suzukii.*
34) The transgenic insect as defined in embodiment 32 or 33, wherein the expression cassette or expression construct is integrated by random stable integration into the genome or by targeted stable integration into the genome, wherein preferably the *piggyBac* system is used for random stable integration, and wherein preferably Recombinase-Mediated Cassette Exchange or a CRISPR/Cas-based system is used for targeted stable integration.
35) The transgenic insect as defined in any one of embodiments 32-34, comprising two copies of an expression cassette according to any one of embodiments 1-19, or of an expression construct according to any one of embodiments 20-29.
36) Use of a transgenic insect according to any one of embodiments 32-35 for controlling reproduction in an insect population of interest, wherein the transgenic insect is capable of interbreeding with insects of the population of interest, preferably wherein the insects are crop pest insects or disease transmitting insects.
37) A method of controlling reproduction in an insect population of interest, comprising the steps of:
   (i) providing a plurality of insects according to any of embodiments 32-35 capable of interbreeding with the insects of the population of interest,
   (ii) optionally selecting suitable individual insects from the plurality, and
   (iii) allowing the insects of step (i) or (ii) to interbreed with insects of the population of interest,
   optionally wherein the insects of step (i) or (ii) are released in an area where reproduction control of insects of the population of interest is desirable.
38) The method of embodiment 37, wherein in step (i) the insects are propagated under conditions, wherein the promoter element, such as a developmental-stage specific promoter element, is repressed.
39) The method of embodiment 37 or 38, wherein in step (ii), male insects are selected from the plurality.
40) A process of generating a transgenic insect comprising the step of introducing an expression cassette according to any one of embodiments 1-20, or an expression construct according to any one of embodiments 21-30, into an insect.
41) The process of embodiment 40, wherein the expression cassette or the expression construct is introduced into the insect by random stable integration into the germline genome or by targeted stable integration into the genome, wherein preferably the *piggyBac* system is used for random stable integration, and wherein preferably Recombinase-Mediated Cassette Exchange or a CRISPR/Cas-based system is used for targeted stable integration.
42) The process of embodiment 40 or 41, further comprising the step of screening hatched G₀ larvae for expression of the expression cassette or the expression construct, preferably wherein the hatched G₀ larvae can be screened for the presence of a fluorescent marker.
43) The process of any one of embodiments 40-42, further comprising the step of establishing independent homozygous strains by single pair inbreeding for successive generations, preferably wherein interbreeding is carried out by backcrossing of G₀ adults with WT males or virgin females.
44) A nuclear localization signal sequence from the *D. suzukii* transformer gene having the amino acid sequence shown in SEQ ID NO. 1.
45) The nuclear localization signal sequence of embodiment 44, wherein said nuclear localization signal sequence is fused C-terminally to an expressed protein.
46) The nuclear localization signal sequence of embodiment 44, wherein the protein is a lethal protein, more preferably a pro-apoptotic gene product.
47) The nuclear localization signal sequence of embodiment 44, wherein the protein is Cas9 protein, transposase, or recombinase.

The invention will be further illustrated by the following figures, sequences and examples, which are not intended to limit the scope of the invention, which is only defined by the claims.

### Description of the Figures

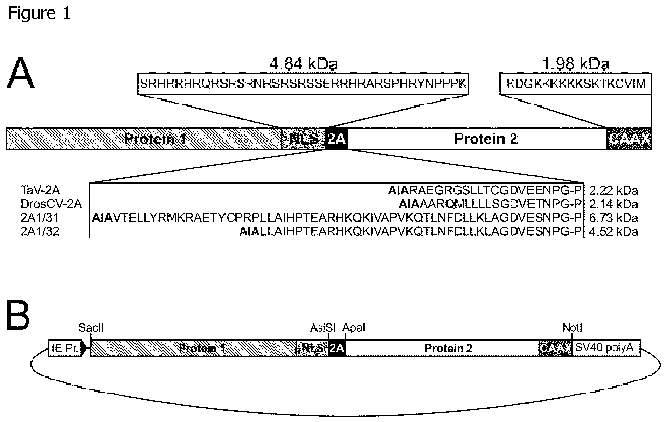

Figure 1 Schematic drawing of bicistronic cassettes containing a 2A-peptide sequence
   (A) Two proteins (protein 1 and protein 2), both containing a localization sequence for different cellular regions, are separated by one out of four different 2A peptide sequences (SEQ ID NO 3-6). Protein 1 and protein 2 are either EGFP or DsRed. NLS is a nuclear localization signal isolated from the *D.suzukii* transformer gene (SEQ ID NO.1). CAAX is a poly-Lysine-CAAX membrane tag (SEQ ID NO.2). Amino acid sequences for NLS, CAAX, and the four different 2A peptides are shown here, are listed in the Description of Sequences, and the Sequence listing (SEQ ID NO. 1-6). The AIA-sequence N-terminal of every 2A peptide is derived from an artificial AsiSI restriction site inserted for cloning purposes. (B) Schematic drawing of pIE-4 derived vectors used for cell culture experiment. Bicistronic cassettes for EGFP-NLS (or DsRed-NLS) and DsRed-CAAX (or EGFP-CAAX), separated by one out of four 2A peptide sequences, are cloned behind the IE promoter of the pIE-4 vector. Unique restriction sites used for cloning are indicated.

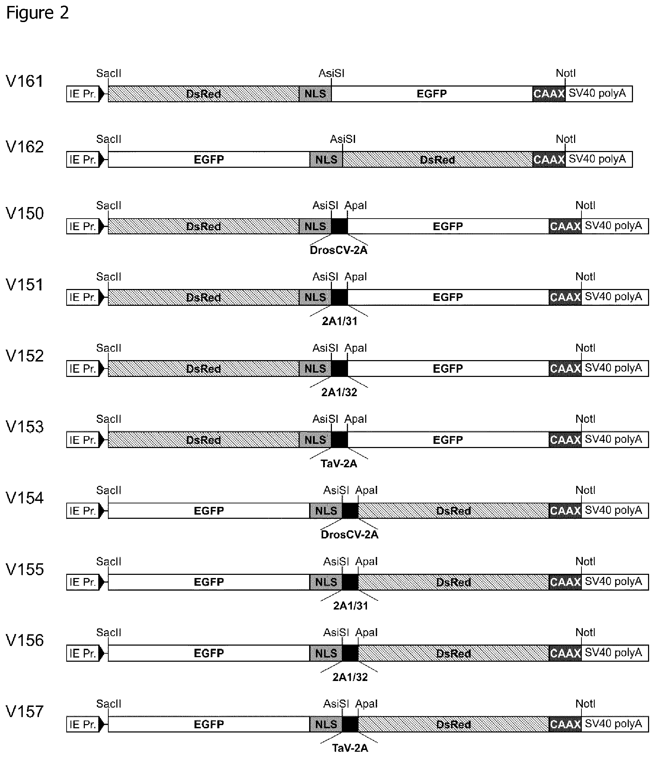

Figure 2 Schematic representation of 2A peptide containing cassettes used for cell culture experiments
   Cassettes used in cell culture experiments are called V150-V157, V161, and V162. All cassettes encode two proteins, EGFP and DsRed as protein 1 or protein 2, respectively. All cassettes are under the pIE-4 promoter. All cassettes contain a SV40 polyA C-terminus. Cassettes V150-157 contain one out of four 2A peptide sequences (SEQ ID NO. 3-6) between protein 1 and protein 2 and are bicistronic. Cassettes V161 and V162 serve as control cassettes as they do not contain a 2A peptide sequence between protein 1 and protein 2. In all cassettes, protein 1 contains an endogenous nuclear localization sequence (NLS) from the *Drosophila suzikii* transformer gene as a C-terminal tag (SEQ ID NO.1). In all cassettes, protein 2 contains a polybasic Lysine rich motif combined with a CAAX motif for membrane localization as a C-terminal tag (SEQ ID NO. 2). Cassettes V150-V153 and V161 contain DsRed as protein 1 and EGFP as protein 2. Cassettes V154-V157 and V162 contain EGFP as protein 1 and DsRed as protein 2. Unique restriction sites used for cloning are indicated.

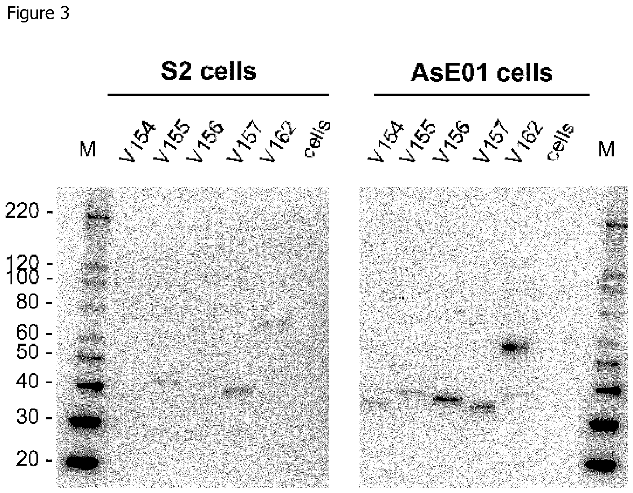

Figure 3 Western Blots to assess cleavage ability of the 2A peptide sequence containing cassettes tested in cell culture
   Cleavability of the 2A peptide sequence of cassettes V154-V157 and V162 was tested in *Drosophila* Schneider S2 cells and *Anastrepha suspensa* cell line UFENY-AsE01. Cassettes V154-V157 were cleaved correctly and efficiently, as demonstrated by the single band signals detected by an anti-GFP antibody (AB290). Protein size varies due to the variable length of the 2A peptide cleavage site. The control cassette V162, not containing a 2A peptide sequence, was not cleaved and shows a fusion protein of EGFP and DsRed of approximately double the size. Non-transfected cells were loaded labelled as "cells" as a control. M refers to the size marker; numbers listed next to the blot refer to protein size in kDa.

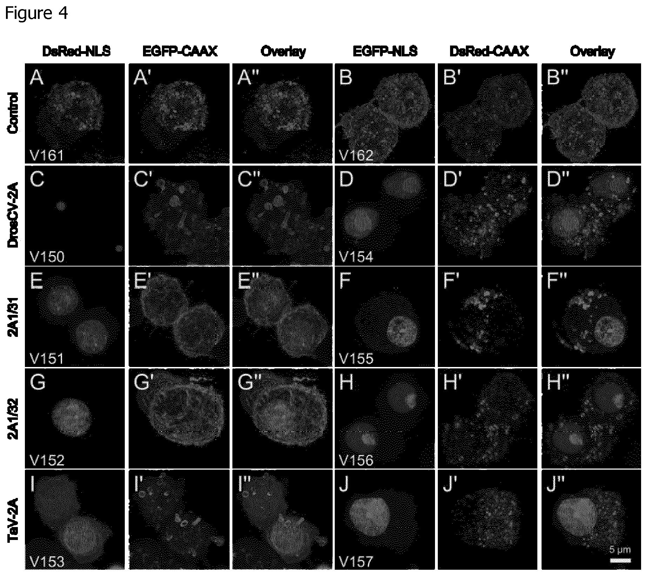

Figure 4 Confocal Microscopy analysis of protein expression and localization using 2A peptide cleavable cassettes in AsE01 Tephritid cells
   All cassettes depicted in Figure 2 (V150-157, V161, and V162) were analysed. Control panels A-A" and B-B" (corresponding to cassettes V161 and V162) expressed an EGFP-DsRed fusion protein and showed co-localization of the two fluorophores in membrane regions. Weaker or undetectable DsRed in some areas might be due to longer maturation time of DsRed. The 2A peptide containing cassettes (panels C-J" corresponding to cassettes V150-V157) show EGFP and DsRed localisation in independent cellular compartments, due to ribosomal skipping during the translation process. The EGFP-CAAX protein (C', E', G', and I') was rather homogenously localized to the cell membrane. DsRed-CAAX (D', F', H' and J'), in contrast, was localized in spotted clusters. In the cassette containing the DrosCV-2A peptide (V150, C-C"), the nuclear DsRed mostly showed subnuclear agglomerations of strong fluorescence instead of DsRed distribution among the entire nuclear region. These cells often showed a membrane 'blebbing' effect in the EGFP channel. A similar, but weaker phenotype could be detected for the TaV-2A containing cassette V153 (I-I").

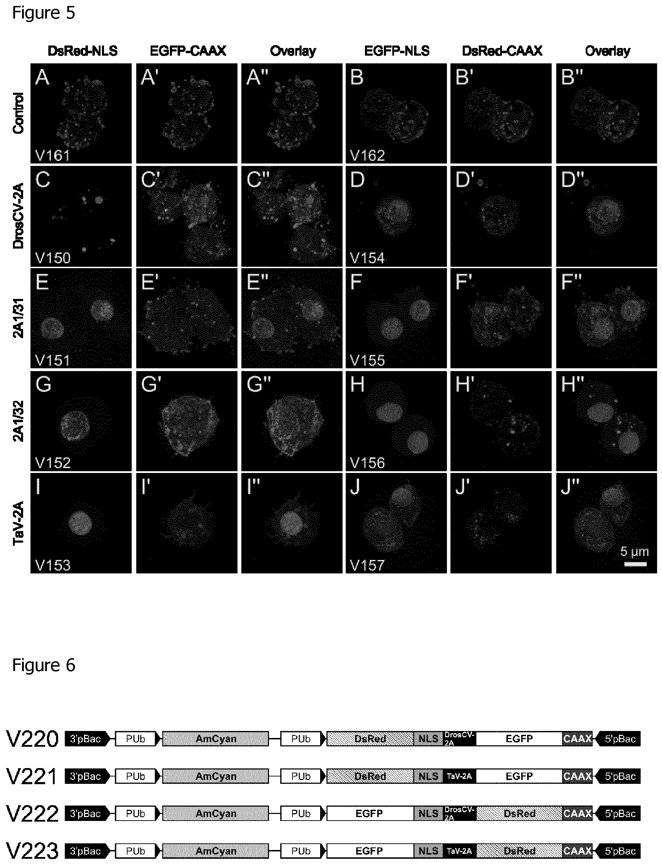

Figure 5 Confocal Microscopy analysis of protein expression and localization using 2A peptide cleavable cassettes in *Drosophila* Schneider S2 cells
   All cassettes depicted in Figure 2 (V150-157, V161, and V162) were analysed. Control cassettes V161 and V162 (panels A-A" and B-B", respectively) expressed an EGFP-DsRed fusion protein and showed co-localization of the two fluorophores. This co-localization occurred in an irregular pattern in the region of the cell membrane and the putative nuclear membrane. The 2A peptide containing cassettes (panels C-J" corresponding to cassettes V150-V157) show EGFP and DsRed localisation in independent cellular compartments, due to ribosomal skipping during the translation process. Some of those cassettes display additional effects. In the DrosCV-2A containing cassette V150 (panel C-C"), DsRed-NLS was usually not homogenously distributed in the nucleus but rather showed strong accumulation in small vesicular structures with strong fluorescence. These cells also usually vesicular structures around the cell membrane in the EGFP channel. In contrast to EGFP-CAAX, DsRed-CAAX (V154-V157) showed a clustered, spotty localization in the cell membrane.
Figure 6 Schematic drawings of vectors used for germline transformation
   Schematic drawings of vectors used for germline transformation to obtain transient and transgenic expression of fluorescent proteins mediated by 2A peptides in *Drosophila suzukii.* Size of the boxes is not to scale. Cassettes used in transient and transgenic expression are referenced as V220-V223. All cassettes encode two proteins, EGFP and dsRed as protein 1 or protein 2 and are separated by a 2A peptide sequence, thus all cassettes are bicistronic. All cassettes are cloned behind a *Drosophila melanogaster* PUb promoter. V220-V223 cassettes also contain an AmCyan gene under control of the PUb promoter as a transformation marker. Poly-A signals are not indicated in this drawing. In V220-V223 cassettes, protein 1 contains an endogenous nuclear localization sequence (NLS) from the *Drosophila suzikii* transformer gene as a C-terminus (SEQ ID NO. 1). In V220-V223 cassettes, protein 2 contains a poly-Lysine-CAAX membrane for membrane localization as a C-terminus (SEQ ID NO. 2). Cassettes V220 and V221 contain DsRed as protein 1 and EGFP as protein 2. Cassettes V222 and V223 contain EGFP as protein 1 and DsRed as protein 2. DsRed and EGFP, are separated by one out of two different 2A peptide (DrosCV-2A or TaV-2A). Cassette V220 and V222 contain the DrosCV-2A peptide sequence (SEQ ID NO. 4) between protein 1 and protein 2; cassettes V221 and V223 contain the TaV-2A peptide sequence (SEQ ID NO. 3) between protein 1 and protein 2.

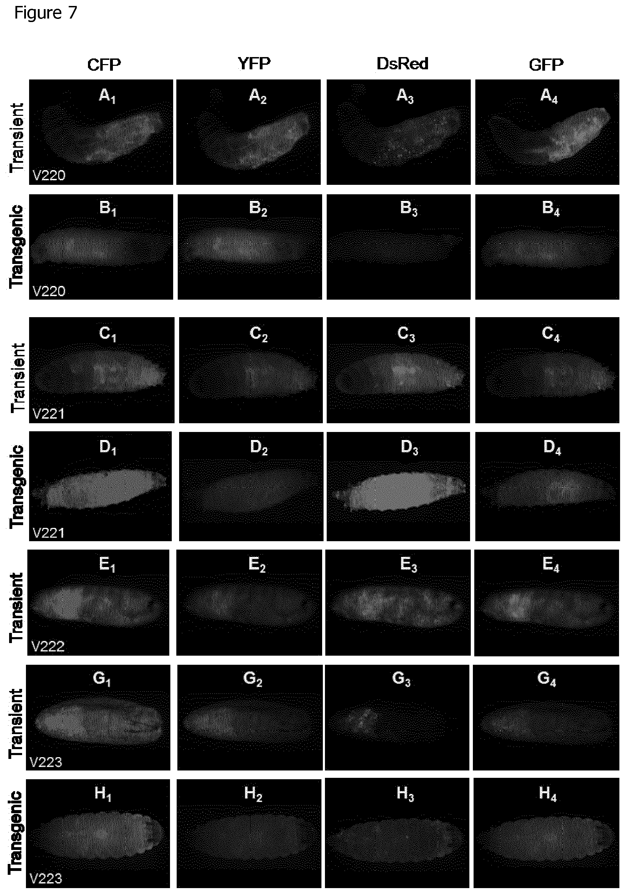

Figure 7 Confocal Microscopy analysis of protein expression and localization using 2A peptide cleavable cassettes in transiently transfected *Drosophila* *suzikii* G₀ larvae and transgenic heterozygous larvae
   Confocal Microscopy analysis of transient and transgenic protein expression and localization. All cassettes depicted in Figure 6 (V220-V223) were analysed. DmPUb is an active and constitutive promoter in *D. suzukii.* Transient expression for AmCyan (A1, C1, E1 and G1), EGFP (A2, C2, E2 and G2), DsRed (A3, C3, E3 and G3) and overlay (A4, C4, E4 and G4), as well as whole body transgenic expression for AmCyan (B1, D1, and H1), EGFP (B2, D2, and H2), DsRed (B3, D3, and H3) and overlay (B4, D4, and H4), were observed. Both DrosCV-2A and TaV-2A worked efficiently to co-express EGFP and DsRed in injected (G₀) and transgenic (heterozygous) flies. The fluorescent intensity of AmCyan is different among transgenic strains, possibly due to the position effect. The fluorescent intensity of EGFP or DsRed are also very different among transgenic strains, which may be subjected to both genomic and cassette position. The third instar larvae from V220 (M1m1), V221 (M3m1) and V223 (M2m1) and a Leica M205FA with the filter sets CFP for AmCyan, YFP for EGFP, TxRed for DsRed and GFP for overlay, were used for imaging.

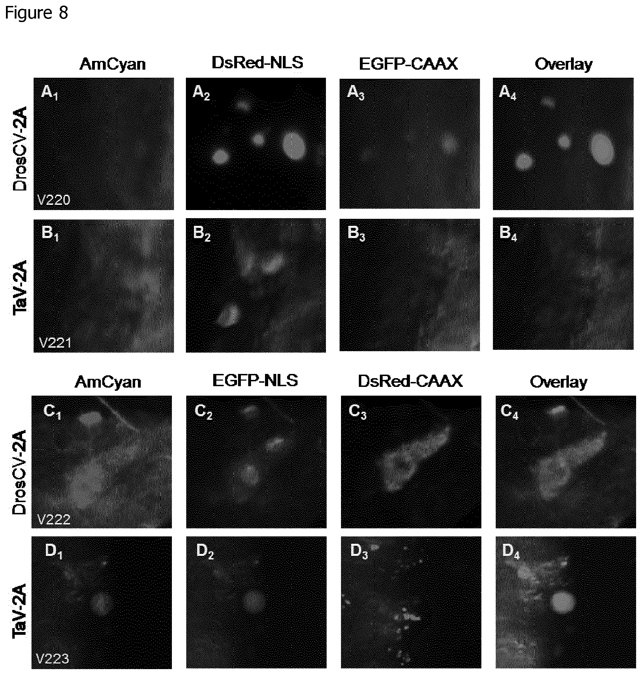

Figure 8 Confocal Microscopy analysis of transient protein expression and localization using 2A peptide cleavable cassettes in gut tissue from injected 3^{rd} instar larvae (G₀) of *Drosophila suzikii*
   Cassettes V220-V223, depicted in Figure 6, were analysed. The AmCyan has no localization signals and is homogenously distributed in the fly tissue (A1, B1, C1 and D1). Both EGFP and DsRed from cassettes V220-V223 were expressed using DrosCV-2A or TaV-2A (A4, B4, C4 and D4). DsRed-NLS (A2 and B2; cassettes V220 and V221) and EGFP- NLS (C2 and D2; cassette V222 and V223) were always localized to the cell nucleus, as expected. The EGFP-CAAX (A3 and B3; cassettes V220 and V221) was rather homogenously localized to the cell membrane, while DsRed-CAAX (C3 and D3; cassettes V222 and V223) was localized in spotted clusters. Gut tissue from injected 3rd instar larvae (G0) was used for imaging.

### Description of the Sequences

| **SEQ name** | **SEQ ID NO** | **Sequence** |
|---|---|---|
| | | (amino acid sequences from N to C-terminus or nucleotide sequences in 5' to 3' direction) |
| NLS | 1 | SRHRRHRQRSRSRNRSRSRSSERRHRARSPHRYNPPPK |
| CAAX | 2 | KDGKKKKKKSKTKCVIM |
| TaV-2A | 3 | AIARAEGRGSLLTCGDVEENPGP |
| DrosCV-2A | 4 | AIAAARQMLLLLSGDVETNPGP |
| 2A1/31 | 5 | AIAVTELLYRMKRAETYCPRPLLAIHPTEARHKQKIVAPVKQTLNFDL LKLAGDVESNPGP |
| 2A1/32 | 6 | AIALLAIHPTEARHKQKIVAPVKQTLNFDLLKLAGDVESNPGP |
| MFS8 | 7 | AAGATTAGCGACGCTGCTG |
| P39 | 8 | CTGCGCGCGATCGATAGTTCTTTGCCTGTATTTCAG |
| P40 | 9 | TTACTTTTATCTAATCTAGACATAGCTCTATAAGATGTGCTCC |
| P180 | 10 | CTACGTGGAAGTGGAAGAAGAAG |
| P191 | 11 | CAGTTGCAAGTTGACACTGG |
| P225 | 12 | TATCCGCGGATGGTGAGCAAGGGCGAGGAG |
| P226 | 13 | TATGCGGCCGCCTACGCGATCGCCTTTGGCGGCGGATTGTACCG |
| P227 | 14 | TGCCGTCCTCCTTCTTAAC |
| P260 | 15 | AGCAGGCACAGAAGGCATCGC |
| P261 | 16 | ATGCCTTCTGTGCCTGCTCTTGTACAGCTCGTCCATGC |
| P262 | 17 | TATCCGCGGATGGCCTCCTCCGAGGACGTCA |
| P263 | 18 | ATGCCTTCTGTGCCTGCTCAGGAACAGGTGGTGGCGGCC |
| P264 | 19 | ACATCCGCGGGGGCCCATGGCCTCCTCCGAGGACGTC |
| P265 | 20 | ACATGCGGCCGCTTACATGATAACACACTTGGTTTTTGACTTCTTC TTCTTTTTCTTACCATCCTTCAGGAACAGGTGGTGGCGGC |
| P266 | 21 | ACATCCGCGGGGGCCCATGGTGAGCAAGGGCGAGGAGC |
| P267 | 22 | ACATGCGGCCGCTTACATGATAACACACTTGGTTTTTGACTTCTTC TTCTTTTTCTTACCATCCTTCTTGTACAGCTCGTCCATGCCGAG |
| P362 | 23 | ACATGCGATCGCGATGGTGAGCAAGGGCGAGGAGC |
| P363 | 24 | ACATGCGATCGCGATGGCCTCCTCCGAGGACGTC |
| P392 | 25 | AACTAGTCGCGAGGCCTGCAGATCTATGGTATATATACACATATT CGATC |
| P393 | 26 | CTTCTTCTTCCACTTCCACGTAGGCCTCCTCCGAGGACGTCATC |
| P394 | 27 | GGCTGATTATGATCTAGAGTCGCGGCCGCCTACGCGATCGCCTTT G |
| P395 | 28 | ATGCCCTGAGGTTGTCGCCGGAACGCAGCGAC |
| P396 | 29 | TAGCAGATCTGGCCGGCCGCGGACGCGTTGGATCCCCGATCTGCA TTTTGG |
| Cctransformer-I intron | 30 | GTAATTTTAAAAGCATATTTTTTTCTTTGAAATTCATAAGTTATCA ATTATCGATGGAAATGTATTCTATGGAGAACGTTTTACCCGATGA ATGGGTGCAAAAATTATTTTACCTTCAAATCTACAATCAACACACG CTAACTTTTGTGACTTGATCAACTCTCACCTGGAAAAGCAACCAAC TACAATCAACATTCTATGGGATAATCGACAAGTGAGTAAAATTATA GCCGGACCTCTTAGTACAGTGTATTTAAAAGGGGAATAATATTCT ATCAATAGGAATAAAAATAAGGTCAGCAGCCATGACTTTTCCATCA TTTTGAATATACCTTATTTGTTTCGGGATTAATTGGGGGTCGGAA ATCCTCTTGAATTCAGAAACGGGAACCGGAGGAAGGTGCCGGTCT TTCAGAAAGCTGTGAAAAATACCAACATTTCTGCTGCCAAGAGCTC AATAAGAAGTTTCAAAAATTGTCTTGGATGTTGCAGCTGTGGCTG CTAAGTAATAAGACATCTATTAGTATCTAGATTTGTTAGACCATTT AACATAGTGTTTTAAACGATGGGGTTAATAGATGAGGGTTAAGAA |
| | | GCTAGTTATATTACTGTTGCTGTAACGCCTTCAATTGTCGGTTACA GAGCAAACATTATTGAATGTTAATGTAAAGAGTTTATTTGTTTTCT AGTAAACATATAGCGATTGGTTAGTAATCACTAATAGAAATTTTTC ATAAGTATCAAAAAAGTAAACCTCTTTTTCAGTCTATGTAATAAGT AAACCAAGGAAAGGGAAAATATCTACAATCAACAAGCCATTGTTG CAGCAACAAAGCAACTGAAACTACAATCAACATTCAATAAACTTGG GTAATTTGGAATTTAATTCTCTGGGACACCTGTGGATTACAACAAT CAACTCGAAACTTATTATACAATGTAAATAAAAATTGATATGCATA CATGAAGATCAAGTGAAATTCCATTTAGAATCAATTTTTTTCGAAT ATTAAGTTTCTTGCTTTAATTTATCTGAAAGTAAATAGACATTCCA AATTCAAGTTAACAAATTAATAATGAATTGACTAGTGATTTTTAAG AGAAAAAGATAAGATTTAAAAAAGGAAAGCCTTTCTTGATAAATTT TTGAACCACTTTATGCCGTTTCAATCATAAAAACTTTTAAGAACAC ATGACTGGTAAAATTAATTTAAAACAAATTTAAATTTCCAACGTAA CATTCAACAAAAATGGTGAAAACTATCACGGAAATTGTTAATATTA ATATGTCCCAAAAATAGCCTTTGTATGTATATGATACTAATCCATA CATCTATGGTATCTATAG |

### Examples

The following examples are meant to further illustrate, but not limit, the invention. The examples comprise technical features, and it will be appreciated that the invention relates also to combinations of the technical features presented in this exemplifying section.

### Example 1 - Construction of bicistronic expression cassettes containing a 2A peptide sequence for cell culture experiments

Several multi-gene expression cassettes were created (*D. suzukii* transgenesis system). For multi-gene expression, two different proteins (protein 1 and protein 2), both containing a localization sequence for different cellular regions, were separated by one out of four different 2A peptide sequences (Figure 1). Protein 1 and protein 2 are either EGFP or DsRed. The following insect virus derived 2A peptides sequences were chosen as follows in this disclosure: the insect virus derived 2A peptide sequences from the *Thosea asigna* virus (TaV) SEQ ID NO. 3, an insect virus derived 2A peptide sequences from *Drosophila* C virus (DrosCV) SEQ ID NO. 4, and two artificial 2A sequences derived from the foot and mouth disease virus (FMDV; 2A1/31 and 2A1/32) SEQ ID NO. 5 and 6, respectively (Donnelly et al., 2001a; incorporated herewith by reference). The amino acid sequences used were identical to the ones previously published (Donnelly et al., 2001a; incorporated herewith by reference). The amino acid sequences are given in the Description of Sequences and in the Sequence listing. The nucleotide sequences were codon optimized for *D. suzukii.* Each test cassette consisted of a gene for an enhanced green fluorescent (EGFP) and a red fluorescent protein (DsRed), divided by a 2A peptide sequence. These peptide sequences are relatively small and increase the size of the first protein by 2.22 to 6.73 kDa. Both *EGFP* and *DsRed* genes contained either a nuclear localization signal (NLS), which has been isolated from the *D. suzukii transformer* gene or a poly-Lysine-CAAX membrane tag (Hancock et al., 1991; incorporated herewith by reference) (SEQ ID NO. 1 and 2, respectively). These localization sequences increase the size of the proteins by another 4.84 and 1.98 kDa for the NLS sequence and the poly-Lysine-CAAX tag, respectively. Combination of two tags with two different proteins allows for the discrete subcellular localization of both fluorophores. The combinations of EGFP and DsRed fluorophores with NLS or poly-Lysine-CAAX membrane tag will furthermore be referred to as EGFP-NLS, EGFP-CAAX, DsRed-NLS, and DsRed-CAAX. Control cassettes (V161 and V162) were created identically to the 2A peptide containing cassettes but did not contain any 2A sequences, hence coding for fusion proteins of the two fluorophores. Expression of the cell culture cassettes was driven by the *immediate early* promoter of the pIE-4 vector (Novagen) via *Sac*II and *Not*I restriction sites, resulting in the cassettes V150-V157, V161 and V162 (Figure 2). Each of the four 2A peptides was constructed in combination with EGFP-NLS and DsRed-CAAX or EGFP-CAAX and DsRed-NLS, respectively. To avoid any potential interference of the C-terminal membrane tag with the 2A peptide sequence, the CAAX-motif was always placed on the C-terminus of the bicistronic cassette.

The *D. suzukii transformer* (*Dz-tra*) gene was isolated (Schwirz, Yan, Schetelig, unpublished data) and the putative *Dz-tra* NLS signal identified based on sequence similarity to the NLS signal from *D. melanogaster,* which has been used as an NLS signal in the *pRed H-Stinger* cassette (Barolo et al., 2004; Hedley et al., 1995; each incorporated herewith by reference). *Dz-tra* NLS was amplified by PCR from vector M1720_pCR4-Dz-transformer_female_transcript using the P260 and P226 primer pair. This amplicon was used during later cloning steps for construction of the transfection vectors for cell culture experiments.

For DsRed-NLS, the *DsRed.T3-gene* was amplified from #1425_*pXLBacII_attP220-PUbDsRedT3* (Schetelig and Handler, 2012; incorporated herewith by reference) using the P262 and P263 primer pair. The amplified *DsRed* and *NLS* fragments were assembled using the Gibson Assembly® Cloning Kit (NEB) following the manufacturer's instructions. The obtained fragment was re-amplified using P262 and P226 primers, and cloned into the pIE4 vector (Novagen) via *Sac*II and *Not*I restriction sites, resulting in the cassette V125_*pIE4_DsRed-NLS.*

For EGFP-NLS, the *EGFP* gene was amplified from #1419_*pXLBacII_PUbEGFP* (Schetelig and Handler, 2012; incorporated herewith by reference) using P225 and P261 primers. The amplified *EGFP* and *NLS* fragments were assembled using Gibson Assembly (New England Biolabs) and the obtained fragment was re-amplified using P225 and P226 primers. The whole fragment was cloned into the pIE4 vector via *Sac*II and *Not*I restriction sites, resulting in V126_*pIE4_EGFP-NLS.*

The CAAX membrane binding motif was designed based on previous work on the membrane targeting of Ras proteins and contains a Lysine-rich polybasic sequence followed by the C-terminal CAAX motif (C= Cysteine, A= aliphatic, X= any amino acid) (Hancock et al., 1990, 1991; incorporated herewith by reference).

*DsRed-CAAX* was amplified from #1425 using P264 and P265 primers. P265 codes for the CAAX membrane tag region. The amplified fragment was cloned into the pIE4 vector via *Sac*II and *Not*I restriction sites, resulting in V123_*pIE4_DsRed-CAAX.*

*EGFP-CAAX* was amplified from #1419 using P266 and P267 primers. P267 codes for the CAAX membrane tag. The amplified fragment was cloned into the pIE-4 vector via *Sac*II and *Not*I restriction sites, resulting in V124-*pIE4-EGFP-CAAX.*

The 2A peptide amino acid sequences for TaV-2A, DrosCV-2A, Ta1/31-2A, and Ta1/32-2A (SEQ ID NO. 3-6) were adapted from previously published work (Donnelly et al., 2001a; incorporated herewith by reference). The nucleotide sequences for these peptides were codon optimized for *D. suzukii,* synthesized, and cloned into the pEX cloning vector by Eurofins Genomics (Luxemburg). The 2A peptide containing cassettes were cloned from the respective pEX vectors into the vector V128_*pIE4-SV40* via *Sac*II and *Not*I restriction sites. This resulted in four 2A peptide destination vectors: V142_*pIE4_DrosCV-2A_SV40,* V143_*pIE4_Ta1*/*31-2A_SV40,* V144_*pIE4_Ta1*/*32-2A_SV40,* and V145_*pIE4_TaV-2A_SV40.* To generate *EGFP-NLS_2A_DsRed-CAAX* cassettes, *EGFP-NLS* was cloned in a first step from *V126_pIE4_EGFP-NLS* via *Sac*II and *Asl*SI restriction sites into the 2A-peptide destination vector. In a second step, *DsRed-CAAX* was cloned from V123*_pIE4_DsRed-CAAX* into this vector via the *Apa*I and *Not*I restriction sites. The *DsRed-NLS_2A_EGFP-CAAX* cassettes were cloned in an analogues approach: *DsRed-NLS* was cloned from V125_*pIE4_DsRed-NLS* via *Sac*II and *Asi*SI restriction sites into the 2A-peptide destination vectors. *EGFP-CAAX* was then cloned from *V124_pIE4-EGFP-CAAX* via the *Apa*I and *Not*I restriction sites into the resulting vector. For the control cassette V161_*pIE4_DsRed-NLS--EGFP-CAAX-SV40, EGFP-CAAX* was amplified from V124*_pIE4-EGFP-CAAX* with Primers P362 (P362_AsiSI_EGFP) and MFS8, and cloned into V125_*pIE4_DsRed-NLS* via *Asi*SI and *Not*I restriction sites. For the control cassette V162_*pIE4_EGFP-NLS-DsRed-CAAX-SV40, DsRed-CAAX* was amplified from V123_*pIE4_DsRed-CAAX* with Primers P363 (P363_AsiSI_DsRed) and MFS8, and cloned into V126_*pIE4_EGFP-NLS* via *Asi*SI and *Not*I restriction sites.

### Example 2 - Expression and cleavage of 2A peptide containing bicistronic cassettes assessed by Western Blot analysis in cell culture

The cleaving ability of the bicistronic 2A cassettes was tested in cell culture, and the cell lysates were subjected to Western blot (Figure 3). In the Western blot analysis, single band signals were detected corresponding to the size of the EGFP protein for each of the 2A peptide cassettes, while bands of approximately double the size have been detected for the control cassette (corresponding to the size of the EGFP-DsRed fusion proteins). Slight size differences on the Western Blot between V154, V155, V156, and V157 (EGFP-NLS) compared to V150, V151, V152, and V153 (EGFP-CAAX) can be explained by the presence of the NLS signal (4.84 kDa for NLS versus 1.98 kDa for CAAX) and the various lengths of individual 2A peptide signals (Figure 3).

The 2A peptides used herewith work well for bicistronic expression in *Anastrepha* AsE01 and *Drosophila* S2 cells. All cassettes led to efficient ribosomal skipping and hence production of both independent gene products as shown through Western blot. In the Western blots, single band signals with the EGFP antibody for all tested cassettes were detected.

Cell lysates were resolved by reducing SDS-PAGE and stained with colloidal Coomassie (Carl Roth, Karlsruhe, Germany) or transferred to a polyvinylidene difluoride (PVDF) membrane (Merck Millipore) using the TransBlot Turbo Transfer System (BioRad). The membrane was blocked overnight in Tris-buffer saline containing 0.1% Tween 20 (TBST) and 2.5% milk (Sigma) and washed (3x 10 min) in TBST prior the 2 hour incubation at room temperature with commercially available polyclonal antibody against EGFP (AB290; ABCAM) diluted 1:2000 in PBST containing 2.5% milk. The membrane was washed with TBST (3x 10 min) and incubated with the secondary goat anti-rabbit whole IgG horseradish peroxidase (HRP) conjugated antibody (Dianova, Hamburg, Germany; 1:5000 in TBST containing 2.5% milk) for 1 h at room temperature. The readout was facilitated using Lumi-Light^{PLUS} Western Blotting Substrate (Roche, Mannheim, Germany) and the resulting signal was visualized using a VersaDoc Imaging System (BioRad).

### Example 3 - Expression and cleavage of bicistronic cassettes in insect cell culture assessed by confocal microscopy

Fluorescence microscopy was used to confirm that all of the 2A peptide-containing cassettes led to cleavage of the bicistronic cassette and a distinct subcellular localization of the nuclear and membrane-tagged fluorescent proteins, respectively, in both AsE01 Tephritid cells (Figure 4) and *Drosophila* S2 cells (Figure 5). This indicates discrete and consecutive production of both proteins and hence functional ribosomal skipping during translation. The cells transfected with the control cassettes V161 and V162, in contrast, showed co-localization of EGFP and DsRed and EGFP-DsRed was most likely present as a fusion protein. In cells transfected with the control cassettes, regions of green fluorescence were missing and only a low red fluorescence level could be detected (Figure 4). This phenomenon might be due to longer maturation time of the DsRed protein compared to EGFP and the initial green fluorescence of immature DsRed protein (Bevis and Glick, 2002; incorporated herewith by reference). The proteins from control cassettes were not localized to the inner nucleus. The fluorescent proteins from control cassettes were present in the region of the plasma membrane as well as the nuclear membrane due to the C-terminal CAAX membrane tag. This membrane anchoring might be the reason why these proteins could not be imported into the nucleus.

For V154 and V157, EGFP was not entirely restricted to the nucleus and a considerable amount of EGFP was also found in the membrane region of the cell. However, not all of these EGFP proteins were co-localized to DsRed. Furthermore, in cells transfected with these cassettes, EGFP expression was still strong in the nucleus, while no EGFP was found in the nucleus for the control cassette (V162). These results showed that both fluorophores were independently expressed with all 2A peptides tested.

The *A. suspensa* cell line UFENY-AsE01 (AsE01) (Shi and Lawrence, 1999; incorporated herewith by reference) and *Drosophila* S2 cells were grown on Schneider's medium with 10% fetal bovine serum (FBS) and 1% penicillin/streptomycin in closed capped flasks without CO₂ at 27.5°C and 25°C, respectively. For transfection experiments, the Xfectin™ transfection reagent (Takara) was used according to manufactures instructions. A total of 4 x 10⁵ cells (live cell count) were seeded in 35 mm glass-bottom cell culture µ-dishes (ibidi) in a volume of 400 µl. After 3 h of settling time, the cells were transfected using 2 µg of plasmid DNA, 0.6 µl Xfectin®, 37.4 µl Xfectin buffer and 360 µl serum-free Schneider's medium for 4 h. Transfection was stopped by medium exchange (Schneider's with 10% FBS and 1% penicillin/streptomycin) in the volume of 2 ml. Cells were incubated for approximately 14 h at 27.5°C and 25°C for AsE01 and S2 cells, respectively. Cells were then fixated for 15 minutes in 4 % PFA in PBS prior to microscopic analysis, or harvested for total cell protein isolation as described below.

Cell culture analysis was done using an SP5 inverted confocal microscope (Leica Microsystems) equipped with 488 nm and 561 nm laser lines for EGFP and DsRed excitation, respectively. Acquired images were deconvolved using Huygens Essential version 15.05 (Scientific Volume Imaging).

### Example 4 - Construction of bicistronic cassettes and germline transformation with expression cassettes to generate transgenic flies

Several multi-gene expression cassettes were generated for the *in vivo* use in *D. suzukii.* The insect virus derived 2A peptide sequences TaV-2A and DrosCV-2A were chosen for further analysis and the sequence is listed in Figure 1, the description of sequences, and the sequence listing as SEQ ID NO. 3 and 4, respectively.

In order to construct 2A peptide sequence containing vectors for germ line transformation, the 2A peptide containing cassettes from cassettes V150, V153, V154, and V157 (obtained in Example 1) were cloned into a *piggyBac* transformation vector for germline transformation of *Drosophila suzukii.* The vector V204_*pXLBacII-PUbAmCyan_PUb_DsRed-NLS-SV40* served as destination vector for all cassettes used in germ line transformations. Cassettes used in germ line transformation are under the DmPUb promoter, which is an active and constitutive promoter in *D. suzukii.*

The vector V204 was created by cloning a part of the 5' region from *DsRed* together with the SV40 region from V193_*pIE4-Dz-traIntron-DsRed-NLS-SV40* into V198_*pXLBacII-attP-[PUbAmCyan_rev]_PUb_DsRed-NLS* via *Sbf*I and *Bgl*II restriction sites and subsequently flipping the *PUb-AmCyan-SV40* cassette by cutting the vector with the *Bsp119*I restriction enzyme and re-ligating the insert into the open vector. For the construction of V193, the *Dz-tra-Intron* sequence was amplified from V38_*pCR4-Dz-transformer-Genomic* (Schwirz, Yan, Schetelig, unpublished data) using primers P392 and P180. The DsRed-NLS sequence was amplified from V125_*pIE4_DsRed-NLS* using primers P393 and P394. The obtained PCR products were cloned into V128_*pIE4-SV40* via Gibson assembly (New England Biolabs), according to the manufacturer's instructions. V198 was constructed by cloning the *DsRed_NLS* sequence from V125*_pIE4_DsRed-NLS* into V195_*pXLBacII-attP-PUbAmCyan_PUb_MCS* by using *Sac*II and *Bgl*II restriction sites. V195 was cloned by amplifying the PUb-MCSsequence through PCR with primers P395 (Bsu36I_PUb_f) and P396 (BglII_PUb_r) from V131_*pXLBacII-attP-PUbAmCyan_Dz_sry-a_tTA_SV40.* The obtained PCR products were cloned into V131 via *Bgl*II and *Bsu36*I restriction sites. The vector V131 was obtained by exchanging the *PUb-DsRed-SV40* cassette from V84_*pXLBacII-attP-PUbDsRedT3_Dz_sry-a_tTA* for the *PUb-AmCyan-SV40* cassette from AH452_*pXL-BACII_FRT_3xP3DsRed_FRT3_loxN-PUbAmCyan-lox2272* (Häcker et al., 2017; incorporated herewith by reference) via *Bsp119*I restriction cloning. V84 was constructed by cloning a 1800bp fragment from V13-*pSLaf_Dsuz_sry-a_tTA-SV40* into #1425 (Schetelig and Handler, 2012) via the *Asc*I and *Fse*I restriction sites. Vector V13 was cloned by amplifying the *Drosophila suzukii serendipity alpha* promoter fragment from vector V4*_pCR4_Dsuz_sry-a* (Schwirz, Yan, Schetelig, unpublished data) using the primers P39 and P40. The vector #1215_*pSLaf_tTA-SV40_af* (Schetelig et al., 2009; incorporated herewith by reference) was cut with *Eco*RV, and *Xba*I and the *Dz_sry-a* PCR fragment was inserted via GeneArt seamless cloning (Thermo Fisher Scientific) according to the manufacturer's instructions.

For V220_*pXLBacII-PUbAmCyan_PUb_DsRed-NLS-DrosCV-2A_EGFP-CAAX-SV40,* the fragment DsRed-NLS-DrosCV-2A_EGFP-CAAX_SV40 was amplified from the donor vector V150 by PCR with primers P227 and P191. The PCR fragment was cloned into V204 via *Sac*II and *Bgl*II restriction sites. This cloning step exchanged the *DsRed_NLS_SV40* cassette from V204 for the *DsRed-NLS-DrosCV-2A_EGFP-CAAX_SV40* cassette from V150. The other three *piggyBac* transformation vectors V221, V222, and V223, were cloned analogously to V220: For V221_*pXLBacII-PUbAmCyan_PUb_DsRed-NLS-TaV-2A_EGFP-CAAX-SV40,* the fragment *DsRed-NLS-TaV-2A-EGFP-CAAX-SV40* was amplified from the donor vector V153 by PCR with primers P227 and P191. The PCR fragment was cloned into V204 via *Sac*II and *Bgl*II restriction sites. For V222_*pXLBacII-PUbAmCyan_PUb_EGFP-NLS-DrosCV-2A_DsRed-CAAX-SV40,* the fragment *EGFP-NLS-DrosCV-2A_DsRed-CAAX-SV40* was amplified from the donor vector V154 by PCR with primers P227 and P191. The PCR fragment was cloned into V204 via *Sac*II and *Bgl*II restriction sites. For *V223_pXLBacII-PUbAmCyan_PUb_EGFP-NLS-TaV-2A_DsRed-CAAX-SV40,* the fragment *EGFP-NLS-TaV-2A_DsRed-CAAX-SV40* was amplified from the donor vector V157 by PCR with primers P227 and P191. The PCR fragment was cloned into V204 via *Sac*II and *Bgl*II restriction sites. Vector maps and sequences for all cassettes are available upon request.

Wild-type (USA strain) and transgenic *D. suzukii* lines were maintained at 25°C and 60% humidity on a 12 h light: 12 h dark cycle. Flies were anesthetized with CO₂ for screening and setup of crosses. Germ-line transformation was conducted as described previously (Schetelig and Handler, 2013; incorporated herewith by reference). Specifically, eggs were collected over a 30 min period on grape juice agar plates (1 % agar, H₂O to grape juice ratio 7:3), desiccated for 10 min and then overlaid with halocarbon oil 700 (Sigma-Aldrich). A mixture of the *piggyBac* donor cassette (500 ng/µl) and the *phsp-pBac* transposase helper plasmid (200 ng/µl) was injected into WT embryos. Hatched G₀ larvae were screened for transient expression of AmCyan using epifluorescence microscopy. Surviving G₀ adults were then backcrossed to WT males or virgin females with putative G₁ transformant progeny selected by AmCyan fluorescence. Independent homozygous strains were established by single pair inbreeding for successive generations and segregation test analysis of transformants outcrossed to WT flies.

### Example 5 - Expression and cleavage of 2A peptide containing bicistronic cassettes in transient Drosophila suzikii G₀ larvae, in transgenic heterozygous larvae, and in injected 3^{rd} instar larvae (G₀) of Drosophila suzikii

Fluorescence microscopy was used to confirm that all of the 2A peptide-containing cassettes led to cleavage of the bicistronic cassette and a distinct localization of the nuclear and membrane-tagged fluorescent proteins, respectively, in both transient and transgenic expression in *Drosophila suzukii.*

The independently localized transient expression of EGFP and DsRed, mediated by either DrosCV-2A or TaV-2A, was readily detectable at late embryogenesis, and continued to the 3^{rd} instar stage (Figure 7). This also suggests that the 2A peptides are compatible with DmPUb promoter for fast and efficient protein production.

Transgenic lines were successfully generated for each cassette and bred to homozygosity. One independent line was obtained for V220 (M1m1) and V221 (M3m1), respectively, and two independent lines for V223 (M2m1 and F2f1). All transgenic lines presented show constitutive expression of AmCyan, EGFP, and DsRed (Figure 7). The fluorescence intensity of AmCyan is different among transgenic strains, possibly due to the position effect. The fluorescence intensity of EGFP or DsRed is also different among transgenic strains, which may also be subjected to both genomic and cassette position.

Fly tissue analysis was done using an SP5 inverted confocal microscope (Leica Microsystems) equipped with 488 nm and 561 nm laser lines for EGFP and DsRed excitation, respectively. Acquired images were deconvolved using Huygens Essential version 15.05 (Scientific Volume Imaging).

The transient expression of G₀ larvae and the transgenic expression of heterozygous larvae were imaged using a Leica M205FA with the filter sets CFP for AmCyan (ex: 436/20; em: 480/40). YFP for EGFP (ex: 500/20; em: 535/30), TxRed for DsRed (ex: 560/40; em: 610 LP) and GFP for overlay (ex: 470/40; em: 525/50). All settings (exposure time, gain, magnification, etc.) were kept identical for each filter so that the fluorescence intensity could be visually compared on the images.

### Example 6 - Cytotoxic effect of DsRed in cell culture and in transgenic D. suzukii strains

In cell culture experiments and transgenic flies, the membrane tagged CAAX-versions of DsRed (DsRed-CAAX) showed unexpected results. While EGFP-CAAX was always homogenously distributed along the cell membrane, DsRed-CAAX was present in intense clusters that were identifiable as strong fluorescent spots in the cell membrane, interspaced by DsRed free regions (Figures 4 and 5). This was also true for both control cassettes, which contained a C-terminal CAAX-tag (V161 and V162, Figure 4 and 5). Since EGFP was directly coupled to DsRed in the controls, it was also localized in clusters in those cases. The reason for this clustering is unknown, but might be connected to the self-association properties of DsRed, which is an obligate tetramer *in vivo* and *in vitro* (Baird et al., 2000; incorporated herewith by reference). In confocal analysis, the cells containing membrane-bound DsRed often showed extensive membrane-blebbing. Similar to the phenomenon observed in the cell culture tests, for transient expressions the EGFP-CAAX was homogenously localized to the cell membrane, while DsRed-CAAX was localized in spotted clusters (Figure 8).

Concluding from cell culture experiments and fly experiments, the CAAX tag efficiently localized the fluorescent proteins to the cell membrane. When this tag was combined with DsRed, the membrane-bound DsRed protein formed cluster agglomeration with detrimental effects on the cell survival in cell culture systems tested. These effects can be used as a novel, universal effector tool for insect control programs.

### List of references

Adelman, Z.N., Tu, Z., 2016. Control of Mosquito-Borne Infectious Diseases: Sex and Gene Drive. Trends Parasitol., Special Issue: Vectors 32, 219-229. https://doi.org/10.1016/j.pt.2015.12.003
Atkinson, P.W., Pinkerton, A.C., O'Brochta, D.A., 2001. Genetic Transformation Systems in Insects. Annu. Rev. Entomol. 46, 317-346. https://doi.org/10.1146/annurev.ento.46.1.317
Baird, G.S., Zacharias, D.A., Tsien, R.Y., 2000. Biochemistry, mutagenesis, and oligomerization of DsRed, a red fluorescent protein from coral. Proc. Natl. Acad. Sci. 97, 11984-11989. https://doi.org/10.1073/pnas.97.22.11984
Barolo, S., Castro, B., Posakony, J.W., 2004. New Drosophila transgenic reporters: insulated P-element vectors expressing fast-maturing RFP. BioTechniques 36, 436-440, 442.
Bevis, B.J., Glick, B.S., 2002. Rapidly maturing variants of the Discosoma red fluorescent protein (DsRed). Nat. Biotechnol. 20, 83-87. https://doi.org/10.1038/nbt0102-83
Cáceres, C., Cayol, J.P., Enkerlin, W.R., Franz, G., Hendrichs, J., Robinson, A.S., 2002. Comparison of Mediterranean fruit fly (Ceratitis capitata)(Tephritidae) bisexual and genetic sexing strains: development, evaluation and economics., in: Proceedings, Symposium: 6th International Symposium on Fruit Flies of Economic Importance. pp. 6-10.
Daniels, R.W., Rossano, A.J., Macleod, G.T., Ganetzky, B., 2014. Expression of Multiple Transgenes from a Single Construct Using Viral 2A Peptides in Drosophila. PLOS ONE 9, e100637. https://doi.org/10.1371/journal.pone.0100637
Donnelly, M.L.L., Hughes, L.E., Luke, G., Mendoza, H., ten Dam, E., Gani, D., Ryan, M.D., 2001a. The 'cleavage' activities of foot-and-mouth disease virus 2A site-directed mutants and naturally occurring '2A-like' sequences. J. Gen. Virol. 82, 1027-1041. https://doi.org/10.1099/0022-1317-82-5-1027
Donnelly, M.L.L., Luke, G., Mehrotra, A., Li, X., Hughes, L.E., Gani, D., Ryan, M.D., 2001b. Analysis of the aphthovirus 2A/2B polyprotein 'cleavage' mechanism indicates not a proteolytic reaction, but a novel translational effect: a putative ribosomal 'skip.' J. Gen. Virol. 82, 1013-1025. https://doi.org/10.1099/0022-1317-82-5-1013
Franz, G., 2005. Genetic Sexing Strains in Mediterranean Fruit Fly, an Example for Other Species Amenable to Large-Scale Rearing for the Sterile Insect Technique, in: Dyck, V.A., Hendrichs, J., Robinson, A.S. (Eds.), Sterile Insect Technique. Springer Netherlands, pp. 427-451. https://doi.org/10.1007/1-4020-4051-2_16
Gilles, J.R.L., Schetelig, M.F., Scolari, F., Marec, F., Capurro, M.L., Franz, G., Bourtzis, K., 2014. Towards mosquito sterile insect technique programmes: Exploring genetic, molecular, mechanical and behavioural methods of sex separation in mosquitoes. Acta Trop., Biology and behaviour of male mosquitoes in relation to new approaches to control disease transmitting mosquitoes 132, Supplement, S178-S187. https://doi.org/10.1016/j.actatropica.2013.08.015
Goedhart, J., Weeren, L. van, Adjobo-Hermans, M.J.W., Elzenaar, I., Hink, M.A., Jr, T.W.J.G., 2011. Quantitative Co-Expression of Proteins at the Single Cell Level - Application to a Multimeric FRET Sensor. PLOS ONE 6, e27321. https://doi.org/10.1371/journal.pone.0027321
Häcker, I., Ii, R.A.H., Eichner, G., Pilitt, K.L., O'Brochta, D.A., Handler, A.M., Schetelig, M.F., 2017. Cre/lox-Recombinase-Mediated Cassette Exchange for Reversible Site-Specific Genomic Targeting of the Disease Vector, Aedes aegypti. Sci. Rep. 7, 43883. https://doi.org/10.1038/srep43883
Hancock, J.F., Cadwallader, K., Paterson, H., Marshall, C.J., 1991. A CAAX or a CAAL motif and a second signal are sufficient for plasma membrane targeting of ras proteins. EMBO J. 10, 4033-4039.
Hancock, J.F., Paterson, H., Marshall, C.J., 1990. A polybasic domain or palmitoylation is required in addition to the CAAX motif to localize p21ras to the plasma membrane. Cell 63, 133-139. https://doi.org/10.1016/0092-8674(90)90294-0
Handler, A.M., 2016. Enhancing the stability and ecological safety of mass-reared transgenic strains for field release by redundant conditional lethality systems. Insect Sci. 23, 225-234. https://doi.org/10.1111/1744-7917.12245
Handler, A.M., Harrell, R.A., 2001. Polyubiquitin-regulated DsRed marker for transgenic insects. BioTechniques 31, 820, 824-8.
Hedley, M.L., Amrein, H., Maniatis, T., 1995. An amino acid sequence motif sufficient for subnuclear localization of an arginine/serine-rich splicing factor. Proc. Natl. Acad. Sci. 92, 11524-11528.
Horn, C., Wimmer, E.A., 2003. A transgene-based, embryo-specific lethality system for insect pest management. Nat. Biotechnol. 21, 64-70. https://doi.org/10.1038/nbt769
Nishizawa, K., Kita, Y., Kitayama, M., Ishimoto, M., 2006. A red fluorescent protein, DsRed2, as a visual reporter for transient expression and stable transformation in soybean. Plant Cell Rep. 25, 1355-1361. https://doi.org/10.1007/s00299-006-0210-x
Nordin, O., Donald, W., Ming, W.H., Ney, T.G., Mohamed, K.A., Halim, N.A.A., Winskill, P., Hadi, A.A., Muhammad, Z.S., Lacroix, R., Scaife, S., McKemey, A.R., Beech, C., Shahnaz, M., Alphey, L., Nimmo, D.D., Nazni, W.A., Lee, H.L., 2013. Oral Ingestion of Transgenic RIDL Ae. aegypti Larvae Has No Negative Effect on Two Predator Toxorhynchites Species. PLOS ONE 8, e58805. https://doi.org/10.1371/journal.pone.0058805
Ogaugwu, C.E., Schetelig, M.F., Wimmer, E.A., 2013. Transgenic sexing system for Ceratitis capitata (Diptera: Tephritidae) based on female-specific embryonic lethality. Insect Biochem. Mol. Biol. 43, 1-8. https://doi.org/10.1016/j.ibmb.2012.10.010
Pavely, C., Fedorova, M., 2006. Early Food Safety Evaluation for a Red Fluorescent Protein: DsRed2.
Rendon, P., McInnis, D., Lance, D., Stewart, J., Tan, K.-H., others, 2000. Comparison of medfly male-only and bisexual releases in large scale field trials., in: Area-Wide Control of Fruit Flies and Other Insect Pests. Joint Proceedings of the International Conference on Area-Wide Control of Insect Pests, 28 May-2 June, 1998 and the Fifth International Symposium on Fruit Flies of Economic Importance, Penang, Malaysia, 1-5 June, 1998. Penerbit Universiti Sains Malaysia, pp. 517-525.
Robinson, A.S., Franz, G., Fisher, K., 1999. Genetic sexing strains in the medfly, Ceratitis capitata: development, mass rearing and field application. Trends Entomol 2, 81-104.
Ryan, M.D., Donnelly, M., Lewis, A., Mehrotra, A.P., Wilkie, J., Gani, D., 1999. A Model for Nonstoichiometric, Cotranslational Protein Scission in Eukaryotic Ribosomes. Bioorganic Chem. 27, 55-79. https://doi.org/10.1006/bioo.1998.1119
Ryan, M.D., King, A.M.Q., Thomas, G.P., 1991. Cleavage of foot-and-mouth disease virus polyprotein is mediated by residues located within a 19 amino acid sequence. J. Gen. Virol. 72, 2727-2732. https://doi.org/10.1099/0022-1317-72-11-2727
Schetelig, M.F., Caceres, C., Zacharopoulou, A., Franz, G., Wimmer, E.A., 2009. Conditional embryonic lethality to improve the sterile insect technique in Ceratitis capitata(Diptera: Tephritidae). BMC Biol. 7, 4. https://doi.org/10.1186/1741-7007-7-4
Schetelig, M.F., Handler, A.M., 2013. Germline transformation of the spotted wing drosophilid, Drosophila suzukii,. Genetica 141, 189-193. https://doi.org/10.1007/s10709-013-9717-6
Schetelig, M.F., Handler, A.M., 2012. Strategy for enhanced transgenic strain development for embryonic conditional lethality in Anastrepha suspensa. Proc. Natl. Acad. Sci. 109, 9348-9353. https://doi.org/10.1073/pnas.1203352109
Schetelig, M.F., Nirmala, X., Handler, A.M., 2011. Pro-apoptotic cell death genes, hid and reaper, from the tephritid pest species, Anastrepha suspensa. Apoptosis 16, 759-768. https://doi.org/10.1007/s10495-011-0610-4
Shah, P., Ding, Y., Niemczyk, M., Kudla, G., Plotkin, J.B., 2013. Rate-limiting steps in yeast protein translation. Cell 153, 1589-1601. https://doi.org/10.1016/j.cell.2013.05.049
Shi, X., Lawrence, P.O., 1999. An embryonic cell line from the caribbean fruit fly, Anastrepha suspensa (Diptera: Tephritidae). Vitro Cell. Dev. Biol. - Anim. 35, 12-14. https://doi.org/10.1007/s11626-999-0036-2
Szymczak, A.L., Workman, C.J., Wang, Y., Vignali, K.M., Dilioglou, S., Vanin, E.F., Vignali, D.A.A., 2004. Correction of multi-gene deficiency in vivo using a single "self-cleaving" 2A peptide-based retroviral vector. Nat. Biotechnol. 22, 589-594. https://doi.org/10.1038/nbt957
Trichas, G., Begbie, J., Srinivas, S., 2008. Use of the viral 2A peptide for bicistronic expression in transgenic mice. BMC Biol. 6, 40. https://doi.org/10.1186/1741-7007-6-40
Vintersten, K., Monetti, C., Gertsenstein, M., Zhang, P., Laszlo, L., Biechele, S., Nagy, A., 2004. Mouse in red: Red fluorescent protein expression in mouse ES cells, embryos, and adult animals. genesis 40, 241-246. https://doi.org/10.1002/gene.20095
Vreysen, M.J.B., Robinson, A.S., Hendrichs, J., 2007. Area-Wide Control of Insect Pests: From Research to Field Implementation. Springer Science & Business Media.
Wang, Y., Wang, F., Wang, R., Zhao, P., Xia, Q., 2015. 2A self-cleaving peptide-based multi-gene expression system in the silkworm Bombyx mori. Sci. Rep. 5. https://doi.org/10.1038/srep16273
Yan, Y., Scott, M.J., 2015. A transgenic embryonic sexing system for the Australian sheep blow fly Lucilia cuprina. Sci. Rep. 5. https://doi.org/10.1038/srep16090
Zhou, L., Schnitzler, A., Agapite, J., Schwartz, L.M., Steller, H., Nambu, J.R., 1997. Cooperative functions of the reaper and head involution defective genes in the programmed cell death of Drosophila central nervous system midline cells. Proc. Natl. Acad. Sci. 94, 5131-5136.

## Claims

1. A polycistronic expression cassette, comprising in functional linkage two or more coding sequences encoding one or more lethal gene product(s), wherein said coding sequences encoding said lethal gene product(s) are each separated by a coding sequence encoding a self-cleaving peptide sequence, wherein the expression cassette is under the control of a single repressible promoter element.

2. The expression cassette of claim 1, wherein the expression of said lethal gene product(s) is regulated with a developmental-stage specific promoter element and/or a sex-specific intron.

3. The expression cassette of claims 1 or 2, wherein the expression cassette comprises up to twelve coding sequences encoding one or more lethal gene product(s), preferably up to eleven coding sequences, preferably up to ten coding, preferably up to nine coding sequences, preferably up to eight coding sequences, preferably up to seven coding sequences, preferably up to six coding sequences encoding one or more lethal gene product(s), more preferably up to five coding sequences encoding one or more lethal gene product(s), more preferably up to four coding sequences encoding one or more lethal gene product(s), even more preferably up to three coding sequences encoding one or more lethal gene product(s), such as two coding sequences encoding one or more lethal gene product(s).

4. The expression cassette of any one of claims 1-3, wherein at least one of said coding sequences encoding said one or more lethal gene product(s) are one or more pro-apoptotic gene(s) or apoptotic gene(s), in particular wherein said one or more pro-apoptotic gene(s) encoding *head involution defective (hid), grim, michelob_x (mx), reaper (rpr), sickle (skl), NippleDm,* an ortholog thereof, or a functional derivative thereof, preferably *hid,* an ortholog thereof, or functional derivatives thereof, in particular wherein said functional derivative is *hid*^{Ala5}; or wherein said one or more apoptotic gene(s) encode for one or more caspase(s), preferably *dronc,* an ortholog thereof, or a functional derivative thereof; in particular wherein said one or more pro-apoptotic gene(s) or apoptotic gene(s) is/are isolated from Dipterans, preferably from the families *Tephritidae* or *Culicidae,* more preferably *Ceratitis capitata, Aedes aegypti, Drosophila melanogaster, Anastrepha ludens, Anastrepha suspensa, Anopheles gambiae,* or *Drosophila suzukii,* most preferably *Ceratitis capitata, Aedes aegypti,* or *Drosophila melanogaster.*

5. The expression cassette of any one of claims 1-4, wherein at least one of said coding sequences encoding said one or more lethal gene product(s) encodes a DsRed-CAAX fusion protein, preferably wherein in said DsRed-CAAX fusion protein a poly-lysine-CAAX membrane tag is fused C-terminally to DsRed, in particular wherein said poly-lysine-CAAX membrane tag has the amino acid sequence shown in SEQ ID NO. 2.

6. The expression cassette of any one of claims 1-5, wherein said encoded self-cleaving peptide sequence is a 2A viral peptide sequence, in particular wherein said 2A viral peptide sequence is selected from 2A viral peptide sequences of the picornavirus family, preferably from the group consisting of (i) 2A from *Thosea asigna* virus as shown in SEQ ID NO. 3, (ii) 2A from *Drosophila* C virus as shown in SEQ ID NO. 4, (iii) 2A from Foot and mouth disease virus (2A1/31) as shown in SEQ ID NO. 5, and (iv) 2A from Foot and mouth disease virus (2A1/32) as shown in SEQ ID NO. 6.

7. The expression cassette of any one of claims 1-6, wherein the repressible promoter element is a response element of a transactivator, wherein binding of said transactivator to said response element induces expression of said polycistronic expression cassette, and wherein the expression of said transactivator is under the control of a promoter, wherein said promoter is a developmental stage specific promoter or a constitutive promoter, preferably wherein said promoter is a developmental stage specific promoter, and wherein the binding of said transactivator to said response element is repressed in the presence of an exogenous factor, in particular wherein the exogeneous factor is tetracycline or a derivative thereof, preferably wherein the derivative is selected from the group consisting of doxycycline, 4-epidoxycycline, anhydrotetracycline, 4-epi-oxytetracycline, chlorotetracycline, and cyanotetracycline.

8. The expression cassette of any one of claims 1-7, wherein at least one of said coding sequences encoding said one or more lethal gene product(s) encoding a fusion protein comprising a C-terminal nuclear localization sequence.

9. The expression cassette of claim 8, wherein said nuclear localization sequence has the amino acid sequence shown in SEQ ID NO. 1 from the *D. suzukii* transformer gene.

10. The expression cassette of any one of claims 1-9, which additionally comprises a sex-specific intron positioned at (i) any position between the transcription start site and the first coding sequence of a lethal gene product, or (ii) within the first coding sequence of a lethal gene product, in particular wherein the sex-specific intron is the intron of the sex determination gene transformer *(Cctransformer-I* intron), preferably the Cctransformer-I intron having the sequence shown in SEQ ID NO. 30.

11. An expression construct, comprising an expression cassette according to any one of claims 1-10, and further comprising a second expression cassette, wherein said second expression cassette comprises one coding sequence or in functional linkage two or more coding sequences expressed polycistronically, in particular wherein said second expression cassette encodes one or more sex-determining gene(s), preferably male-determining gene(s), more preferably M-factor on the Y-chromosome of *Ceratitis capitata,* the *nix* gene from *Aedes aegypti,* the *GUY1* gene from *Anopheles stephensi,* or the *mdmd gene* from *Musca domestica.*

12. A transgenic insect comprising an expression cassette according to any one of claims 1-10, or an expression construct according to claims 11, in particular wherein the insect belongs to Dipterans, preferably crop pest flies or disease transmitting flies belonging to the families *Tephritidae* or *Culicidae,* preferably to the genus *Drosophilidae, Anastrepha,* or *Aedes,* more preferably of the group *D. suzukii, D. melanogaster, Anastrepha suspensa,* or *Aedes aegypti,* most preferably wherein the insect is a *D. suzukii.*

13. The transgenic insect as defined in claim 12, wherein the expression cassette or expression construct is integrated by random stable integration into the genome or by targeted stable integration into the genome, wherein preferably the *piggyBac* system is used for random stable integration, and wherein preferably Recombinase-Mediated Cassette Exchange or a CRISPR/Cas-based system is used for targeted stable integration.

14. Use of a transgenic insect according to claim 12 or 13 for controlling reproduction in an insect population of interest, wherein the transgenic insect is capable of interbreeding with insects of the population of interest, preferably wherein the insects are crop pest insects or disease transmitting insects.

15. A method of controlling reproduction in an insect population of interest, comprising the steps of:
(i) providing a plurality of insects according to claim 12 or 13, capable of interbreeding with the insects of the population of interest,
(ii) optionally selecting suitable individual insects from the plurality, and
(iii) allowing the insects of step (i) or (ii) to interbreed with insects of the population of interest,
optionally wherein the insects of step (i) or (ii) are released in an area where reproduction control of insects of the population of interest is desirable, and preferably wherein in step (i) the insects are propagated under conditions, wherein the promoter element, such as a developmental-stage specific promoter element, is repressed; and/or in step (ii), male insects are selected from the plurality.
